# EUROPEAN PATENT APPLICATION

(11) **EP 3 690 028 A1**
(43) Date of publication of application: **05.08.2020**
(21) Application number: 19217072.8
(22) Date of filing: 03.09.2013
(51) Int. Cl.: C12N 5/00, A61L 27/36

(54) **METHODS OF TISSUE GENERATION**

(30) Priority: 04.09.2012 US 201261696479 P
(62) Divisional of application: 13835205.9
(71) Applicant: Anthrogenesis Corporation, Warren, NJ 07059 (US)
(72) Inventor: Bhatia, Mohit, B., Manalapan, NJ 07726 (US); Hariri, Robert, J., Bernardsville, NJ 07924 (US); Hofgartner, Wolfgang, Florham Park, NJ 07932 (US); Wang, Jia-Iun, Cherry Hill, NJ 08003 (US); Ye, Qian, Livingstone, NJ 07039 (US)
(74) Representative: Mewburn Ellis LLP

(57) **Abstract**

Provided herein are methods of generating tissues and organs in vitro or ex vivo comprising depositing cells and ex-tracellular matrix onto a surface, as well as methods of using such tissues and organs. In one embodiment, the cells and ECM used in accordance with the methods for generating tissues (e.g. three-dimensional tissues) and organs described herein and deposited as part of the same composition. In another embodiment, the cells and ECM used in accordance with the methods for generating tissues (e.g. three-dimensional tissues) and organs described herein are deposited as part of different compositions.

## Description

### METHODS OF TISSUE GENERATION

This application claims priority to U.S. provisional patent application No.
61/696,479, filed September 4, 2012, the disclosure of which is herein incorporated by reference
in its entirety.

1.	INTRODUCTION

Provided herein are methods of generating tissues and organs in vitro or ex vivo
comprising depositing cells and/or an extracellular matrix onto a surface, as well as methods of
using such tissues and organs.

2.	BACKGROUND

Bioprinting (e.g., organ printing) is an area of research and engineering that involves
printing devices, such as modified ink-jet printers, that deposit biological material. The
technology involves the rapid creation and release of liquid droplets comprising cells followed
by their precise deposition on a surface. Tissues and organs engineered using basic cellular
materials by means of bioprinting represent a promising alternative to the donor-derived tissues
and organs that are used today in standard transplantation approaches.

3.	SUMMARY

In one aspect, provided herein is a method for generating a tissue (e.g., a three-
dimensional tissue) or an organ comprising depositing cells and/or an extracellular matrix (ECM)
onto a surface in vitro or ex vivo so as to form said tissue. Cells that may be used in accordance
with the methods for generating tissues (e.g., three-dimensional tissues) and organs described
herein are described in Section 4.1.1, below. Tissues and Organs that may be engineered in
accordance with the methods for generating tissues (e.g., three-dimensional tissues) and organs
described herein are described in Section 4.1.2, below. ECM that may be used in accordance
with the methods for generating tissues (e.g., three-dimensional tissues) and organs described
herein is described in Section 4.1.3, below. Surfaces onto which cells, ECM, and/or additional

components may be deposited in accordance with the methods for generating tissues (e.g., three-
dimensional tissues) and organs described herein are described in Section 4.1.4, below.
In one embodiment, the cells and ECM used in accordance with the methods for
generating tissues (e.g., three-dimensional tissues) and organs described herein are deposited as
part of the same composition. In another embodiment, the cells and ECM used in accordance
with the methods for generating tissues (e.g., three-dimensional tissues) and organs described
herein are deposited as part of different compositions. In a specific embodiment, the ECM used
in accordance with the methods for generating tissues (e.g., three-dimensional tissues) and
organs described herein comprises flowable ECM. In another specific embodiment, the cells and
ECM used in accordance with the methods for generating tissues (e.g., three-dimensional tissues)
and organs described herein are deposited as part of different compositions, for example,
wherein the ECM is deposited separate from, e.g., before the deposition of the cells, and/or
wherein the ECM is dehydrated prior to the deposition of the cells. In embodiments where the
ECM is dehydrated, it may later be rehydrated at a desired time, e.g., at the time cells are
deposited onto the surface that the ECM and cells have been deposited on.
In certain embodiments, the cells and ECM used in the methods for forming three-
dimensional tissues in vivo described herein are deposited onto a surface concurrently, before, or
after deposition of one or more additional components, e.g., a growth factor(s), a cross-linker(s),
a polymerizable monomer(s), a polymer, a hydrogel(s), etc. In certain embodiments, the surface
onto which said cells and ECM are deposited is a surface that has been bioprinted in accordance
with the methods described herein.
In certain embodiments, the cells and flowable ECM (as well as additional
components) used in accordance with the methods for generating tissues (e.g., three-dimensional
tissues) and organs described herein are printed onto said surface, e.g., the cells and ECM are
bioprinted. In certain embodiments, the surface onto which said cells and ECM are bioprinted is
a surface that has been bioprinted in accordance with the methods described herein.
In certain embodiments, the cells and/or flowable ECM (as well as additional
components) used in accordance with the methods for generating tissues (e.g., three-dimensional
tissues) and organs described herein are not printed onto said surface, e.g., the cells and ECM are
not bioprinted but, rather, are applied to said surface by a method that does not comprise
bioprinting. In certain embodiments, the cells and/or flowable ECM (as well as additional

components) that are not bioprinted onto a surface are applied to a surface that has been
bioprinted, e.g., the cells and/or flowable ECM (as well as additional components) are applied to
a scaffold, e.g., a synthetic scaffold, such as a synthetic matrix. In a specific embodiment, the
cells and/or flowable ECM (as well as additional components) are applied to only part, e.g., one
side, of the scaffold (e.g., the surface). In another specific embodiment, the cells and/or flowable
ECM (as well as additional components) are applied to all sides of the scaffold, i.e., the entire
scaffold has cells and/or flowable ECM applied to it. In another specific embodiment, the
scaffold is polycaprolactone (PCL).
In certain embodiments, the surface onto which cells, ECM, and/or additional
components may be deposited in accordance with the methods for generating tissues (e.g., three-
dimensional tissues) and organs described herein comprises an artificial surface, i.e., a surface
that has been man-made. In another specific embodiment, the surface onto which cells, ECM,
and/or additional components may be deposited in accordance with the methods for generating
tissues (e.g., three-dimensional tissues) and organs described herein comprises tissue or an organ
(or portion thereof) that has been removed from a subject (e.g., a human subject). In certain
embodiments, the surface of said tissue or an organ that has been removed from a subject may be
decellularized, e.g., treated so as to remove cells from all or part of the surface of the tissue or
organ. In certain embodiments, the surface onto which cells, ECM, and/or additional
components may be deposited in accordance with the methods for generating tissues (e.g., three-
dimensional tissues) and organs described herein is two-dimensional. In certain embodiments,
the surface onto which cells, ECM, and/or additional components may be deposited in
accordance with the methods for generating tissues (e.g., three-dimensional tissues) and organs
described herein is three-dimensional. In a specific embodiment, the surface onto which cells,
ECM, and/or additional components may be deposited in accordance with the methods for
generating tissues (e.g., three-dimensional tissues) and organs described herein is a surface that
has been bioprinted, e.g., bioprinted in accordance with the methods described herein. In a
specific embodiment, the surface is polycaprolactone (PCL).
In another aspect, provided herein are tissues and organs generated using the methods
described herein, as well as methods of using such tissues and organs.

In certain embodiments, the tissues (e.g., three-dimensional tissues) and organs
engineered in accordance with the methods described herein are used in transplantation
procedures, including skin grafts and surgical transplantation procedures.
In certain embodiments, the tissues (e.g., three-dimensional tissues) and organs
engineered in accordance with the methods described herein are used in experimental
procedures, e.g., to assess the effect of a drug or compound on said tissue or organ.
In another aspect, provided herein are compositions comprising cells and ECM (e.g.,
a flowable ECM), wherein said compositions are suitable for use in the methods described
herein.  Also provided herein are kits comprising, in one or more containers, said compositions,
as well as instructions for using said compositions in accordance with one or more of the
methods described herein.
3.1	BRIEF DESCRIPTION OF DRAWINGS
Fig. 1 depicts scaffolds comprising polycaprolactone (PCL) that were bioprinted at
various angles and in such a way that scaffolds of various pore sizes were generated.
Fig. 2 depicts multiple view of bioprinted scaffolds onto which extracellular matrix
(ECM) has been applied to both sides of the scaffold and subsequently dehydrated.
Fig. 3 depicts the results of a cell proliferation assay.  Placental stem cells cultured on
a hybrid scaffold comprising bioprinted PCL and dehydrated ECM proliferate over an 8-day
culture period.
Fig. 4 depicts the results of a cell viability assay.  Placental stem cells cultured on a
hybrid scaffold comprising bioprinted PCL and dehydrated ECM proliferated and remained
viable over an 8-day culture period.
Fig. 5 depicts an intact three-dimensional hybrid scaffold comprising PCL, ECM, and
placental stem cells, each of which were bioprinted as layers (layers of PCL and layers of
ECM/cells).
Fig. 6 demonstrates that placental stem cells distribute throughout three-dimensional
bioprinted scaffolds over a 7-day culture period.
Fig. 7 depicts the results of a cell viability assay.  Placental stem cells bioprinted with
ECM and PCL to form a three-dimensional hybrid scaffold proliferate and remain viable over a
7-day culture period.

Fig. 8 demonstrates that stem cells bioprinted with ECM and PCL to form a three-
dimensional hybrid scaffold spread throughout the ECM in the hybrid scaffolds over a 7-day
culture period.
Fig. 9 depicts the results of a cell proliferation assay. Placental stem cells cultured in
a three-dimensional hybrid scaffold that was generated by bioprinting PCL, ECM, and placental
stem cells proliferate over a 7-day culture period.
Fig. 10 depicts a bioprinted scaffold comprising PCL, placental ECM, and insulin-
producing cells (ß-TC-6 cells).
Fig. 11 depicts the results of a cell proliferation assay. Numbers of insulin-producing
cells (ß-TC-6 cells) in a bioprinted scaffold comprising PCL, placental ECM, and insulin-
producing cells remained steady over a 14-day culture period.
Fig. 12 depicts levels of insulin production from bioprinted scaffolds comprising
PCL, placental ECM, and insulin-producing cells (ß-TC-6 cells).
Fig. 13 depicts levels of insulin production from bioprinted scaffolds comprising
PCL, placental ECM, and insulin-producing cells (ß-TC-6 cells) following exposure to glucose
challenge (A) or under control conditions (B, C).

4.	DETAILED DESCRIPTION

In one aspect, provided herein is a method for generating a tissue (e.g., a three-
dimensional tissue) or an organ comprising depositing cells and extracellular matrix (ECM) onto
a surface in vitro or ex vivo so as to form said tissue. Cells that may be used in accordance with
the methods for generating tissues (e.g., three-dimensional tissues) and organs described herein
are described in Section 4.1.1, below. Tissues and Organs that may be engineered in accordance
with the methods for generating tissues (e.g., three-dimensional tissues) and organs described
herein are described in Section 4.1.2, below. ECM that may be used in accordance with the
methods for generating tissues (e.g., three-dimensional tissues) and organs described herein is
described in Section 4.1.3, below. Surfaces onto which cells, ECM, and/or additional
components may be deposited in accordance with the methods for generating tissues (e.g., three-
dimensional tissues) and organs described herein are described in Section 4.1.4, below.

In a specific embodiment, provided herein is a method for generating a tissue (e.g., a
three-dimensional tissue) or an organ comprising depositing cells and ECM onto a surface in
vitro or ex vivo so as to form said tissue or organ.
In another specific embodiment, provided herein is a method for generating a tissue
(e.g., a three-dimensional tissue) or an organ comprising depositing cells and ECM onto a
surface in vitro or ex vivo so as to form said tissue or organ, wherein said ECM comprises
flowable ECM, and wherein said cells and said flowable ECM are formulated as part of the same
composition. In a specific embodiment, said cells and said ECM are deposited using a
bioprinter. In another specific embodiment, said cells comprise a single type of cell. In another
specific embodiment, said cells comprise more than one type of cell.
In another specific embodiment, provided herein is a method for generating a tissue
(e.g., a three-dimensional tissue) or an organ comprising depositing cells and ECM onto a
surface in vitro or ex vivo so as to form said tissue or organ, wherein said ECM comprises
flowable ECM, and wherein said cells and said flowable ECM are formulated as part separate
compositions. In a specific embodiment, said cells and said ECM are deposited using a
bioprinter. In another specific embodiment, said cells comprise a single type of cell. In another
specific embodiment, said cells comprise more than one type of cell.
In another specific embodiment, provided herein is a method for generating a tissue
(e.g., a three-dimensional tissue) or an organ comprising depositing cells and ECM onto a
surface in vitro or ex vivo so as to form said tissue or organ, wherein said ECM comprises
flowable ECM, and wherein said cells and said flowable ECM are formulated as part separate
compositions. In a specific embodiment, said cells and said ECM are deposited using a
bioprinter. In another specific embodiment, said cells comprise a single type of cell. In another
specific embodiment, said cells comprise more than one type of cell.
In another specific embodiment, provided herein is a method for generating a tissue
(e.g., a three-dimensional tissue) or an organ comprising depositing cells, ECM, and one or more
additional components onto a surface in vitro or ex vivo so as to form said tissue or organ.
In another specific embodiment, provided herein is a method for generating a tissue
(e.g., a three-dimensional tissue) or an organ comprising depositing cells, ECM, and one or more
additional components onto a surface in vitro or ex vivo so as to form said tissue or organ. In a
specific embodiment, said cells, said ECM, and said one or more additional components are

deposited using a bioprinter. In another specific embodiment, said cells comprise a single type
of cell. In another specific embodiment, said cells comprise more than one type of cell. In
another specific embodiment, said cells, said ECM, and said one or more additional components
are formulated as part of the same composition. In another specific embodiment, said cells, said
ECM, and said one or more additional components are formulated as part separate compositions.
In another specific embodiment, said one or more additional components is a growth factor, a
polymerizable monomer, a cross-linker, a polymer, or a hydrogel.
In certain embodiments, the surface onto which cells, ECM, and/or additional
components may be deposited in accordance with the methods for generating tissues (e.g., three-
dimensional tissues) and organs described herein comprises an artificial surface, i.e., a surface
that has been man-made. In another specific embodiment, the surface onto which cells, ECM,
and/or additional components may be deposited in accordance with the methods for generating
tissues (e.g., three-dimensional tissues) and organs described herein comprises tissue or an organ
(or portion thereof) that has been removed from a subject (e.g., a human subject). In certain
embodiments, the surface of said tissue or an organ that has been removed from a subject may be
decellularized, e.g., treated so as to remove cells from all or part of the surface of the tissue or
organ. In certain embodiments, the surface onto which cells, ECM, and/or additional
components may be deposited in accordance with the methods for generating tissues (e.g., three-
dimensional tissues) and organs described herein is two-dimensional. In certain embodiments,
the surface onto which cells, ECM, and/or additional components may be deposited in
accordance with the methods for generating tissues (e.g., three-dimensional tissues) and organs
described herein is three-dimensional. In a specific embodiment, the surface onto which cells,
ECM, and/or additional components may be deposited in accordance with the methods for
generating tissues (e.g., three-dimensional tissues) and organs described herein is a surface that
has been bioprinted, e.g., bioprinted in accordance with the methods described herein. In a
specific embodiment, the surface comprises a synthetic material, e.g., a synthetic polymer. In
another specific embodiment, the synthetic polymer is PCL.
In certain embodiments, the cells and ECM (e.g., a flowable ECM) are not printed
concurrently, but are printed in layers. In a specific embodiment, a layer of cells is printed on a
surface, followed by the printing of a layer of ECM. In another specific embodiment, a layer of
ECM is printed on a surface, followed by the printing of a layer of cells. In certain

embodiments, multiple layers of ECM can be printed on a surface followed by the printing of
multiple layers of cells, and vice versa. Likewise, additional components that are printed
concurrently with, before, or after the printing of cells and/or ECM may be layered among cells
and ECM in accordance with the methods described herein.
In certain embodiments, the cells and ECM (e.g., a flowable ECM) are printed such
that the surface being printed on is wholly covered by both cells and ECM. In other
embodiments, the cells and ECM (e.g., a flowable ECM) are printed such that the surface being
printed on is partially covered by both cells and ECM.
In certain embodiments, the cells and ECM (e.g., a flowable ECM) are printed such
that the surface being printed on is covered by cells in specific, desired areas; and covered by
ECM in specific, desired areas, wherein such specific areas may or may not overlap.
In certain embodiments, the cells and ECM (e.g., a flowable ECM) may be printed
onto a surface three dimensionally. As used herein "three-dimensional printing" refers to the
process of printing such that the print heads of bioprinter move below, above, and around a
three-dimensional surface, e.g., the printer heads are mechanically controlled so as to rotate
along a specified path. As used herein, three-dimensional printing is in contrast to standard
methods of bioprinting that are known in the art, where the printing is performed by starting to
build tissue on a flat/planar/two-dimensional surface.
In one embodiment, ECM is printed on a surface (e.g., a prosthetic or a bone) in vitro
or ex vivo, and cells are later seeded on said surface that comprises ECM using standard cell
culturing approaches. Such a surface may then be transplanted into a subject. In another
embodiment, ECM is printed on a surface (e.g., a prosthetic or bone) in vitro or ex vivo, and said
surface that comprises ECM is transplanted into a subject, wherein cells of subject attach to
and/or grow on said surface.
In a specific embodiment, provided herein is a method for generating a tissue
comprising depositing cells and ECM onto a surface in vitro or ex vivo so as to form said tissue,
wherein said surface comprises a bone having an inner face and an outer face, and wherein a first
cellular composition comprising a first type of cell is printed on said inner face, and a second
cellular composition comprising second type of cell is printed on said outer face. As used herein,
the "inner face" of the bone represents the face of the bone intended to lie against stromal and
muscle tissue, and the "outer face" of the bone represents the face of the bone intended to be

exposed to the exterior of the recipient's body. In accordance with this embodiment, the inner
face may be covered partially or wholly by, e.g., stromal cells, fatty tissue, mesenchymal stem
cells, myocytes, or combinations of the like, and the outer face may be covered partially or
wholly by, e.g., dermal cells. In a specific embodiment, said method additionally comprises the
deposition of one more additional components (e.g., a cross-linker). In another specific
embodiment, said printing is performed three-dimensionally.
In a specific embodiment, provided herein is a method for generating a liver
comprising depositing cells and ECM onto a surface in vitro or ex vivo so as to form said liver,
wherein said surface comprises liver tissue. In a specific embodiment, the liver tissue is obtained
from the subject for which the liver generated is intended to be transplanted. In another specific
embodiment, the liver tissue is not obtained from the subject for which the liver generated is
intended to be transplanted (e.g., the liver tissue is obtained from a living or cadaveric donor). In
another specific embodiment, said cells that are deposited are liver cells (e.g., hepatocytes). In a
specific embodiment, said method additionally comprises the deposition of one more additional
components (e.g., a cross-linker). In another specific embodiment, said printing is performed
three-dimensionally.
In a specific embodiment, provided herein is a method for generating a skin
comprising depositing cells and ECM onto a surface in vitro or ex vivo so as to form said skin,
wherein said surface comprises skin tissue. In a specific embodiment, the skin tissue is obtained
from the subject for which the skin generated is intended to be transplanted. In another specific
embodiment, the skin tissue is not obtained from the subject for which the skin generated is
intended to be transplanted (e.g., the skin tissue is obtained from a living or cadaveric donor). In
another specific embodiment, said cells that are deposited are skin cells (e.g., epidermal cells).
In a specific embodiment, said method additionally comprises the deposition of one more
additional components (e.g., a cross-linker). In another specific embodiment, said printing is
performed three-dimensionally.

4.1 BIOPRINTING

"Bioprinting," as used herein, generally refers to the deposition of living cells, as well
as other components (e.g., a flowable ECM; synthetic matrices) onto a surface using standard or
modified printing technology, e.g., ink jet printing technology. Basic methods of depositing cells

onto surfaces, and of bioprinting cells, including cells in combination with hydrogels, are
described in Warren et al. US 6,986,739, Boland et al. US 7,051,654, Yoo et al. US
2009/0208466 and Xu et al. US 2009/0208577, the disclosures of each of which are incorporated
by reference herein their entirety. Additionally, bioprinters suitable for production of the tissues
and organs provided herein are commercially available, e.g., the 3D-Bioplotter™ from
Envisiontec GmbH (Gladbeck, Germany); and the NovoGen MMX Bioprinter™ from Organovo
(San Diego, CA).
The bioprinter used in the methods described herein may include mechanisms and/or
software that enables control of the temperature, humidity, shear force, speed of printing, and/or
firing frequency, by modifications of, e.g., the printer driver software and/or the physical makeup
of the printer. In certain embodiments, the bioprinter software and/or hardware preferably may
be constructed and/or set to maintain a cell temperature of about 37°C during printing.
In certain embodiments, the inkjet printing device may include a two-dimensional or
three-dimensional printer. In certain embodiments, the bioprinter comprises a DC solenoid
inkjet valve, one or more reservoir for containing one or more types of cells, e.g., cells in the
flowable composition, and/or ECM (e.g., a flowable ECM) prior to printing, e.g., connected to
the inkjet valve. The bioprinter may have 1, 2, 3, 4, 5, 6, 7, 8, 9, 10 or more reservoirs, e.g., one
for each cell type or each ECM used to construct the tissues and organs described herein. The
cells may be delivered from the reservoir to the inkjet valve by air pressure, mechanical pressure,
or by other means. Typically, the bioprinter, e.g., the print heads in the bioprinter, is/are
computer-controlled such that the one or more cell types, and said ECM, are deposited in a
predetermined pattern. Said predetermined pattern can be a pattern that recreates or recapitulates
the natural arrangement of said one or more types of cells in an organ or tissue from which the
cells are derived or obtained, or a pattern that is different from the natural arrangement of said
one or more types of cells.
In certain embodiments, the bioprinter used in the methods provided herein may be a
thermal bubble inkjet printer, see, e.g., Niklasen et al. US 6,537,567, or a piezoelectric crystal
vibration print head, e.g., using frequencies up to 30 kHz and power sources ranging from 12 to
100 Watts. Bioprinter print head nozzles, in some embodiments, are each independently
between 0.05 and 200 micrometers in diameter, or between 0.5 and 100 micrometers in diameter,
or between 10 and 70 micrometers in diameter, or between 20 and 60 micrometers in diameter.

In further embodiments, the nozzles are each independently about 40 or 50 micrometers in
diameter. Multiple nozzles with the same or different diameters may be used. In some
embodiments the nozzles have a circular opening; in other embodiments, other suitable shapes
may be used, e.g., oval, square, rectangle, etc., without departing from the spirit of the invention.
In certain embodiments, an anatomical image of the tissue or organ to be bioprinted
may be constructed using software, e.g., a computer-aided design (CAD) software program. In
accordance with such embodiments, programs can be generated that allow for three-dimensional
printing on a three-dimensional surface that is representative of the structure of the tissue or
organ to be printed. For example, if it is desired to print a bone, an anatomical image of the bone
may be constructed and a program may be generated that directs the printer heads of the
bioprinter to rotate around the three-dimensional bone surface during printing.
In certain embodiments, the methods of bioprinting provided herein comprise the
delivery/deposition of individual droplets of cells (e.g., compositions comprising single cells or
compositions comprising multiple cells) and flowable extracellular matrix (ECM) on a surface.
In certain embodiments, the methods of bioprinting provided herein comprise the
deposition of a single cell type and flowable ECM on a surface. Exemplary cell types that can be
used in accordance with such methods are provided in Section 4.1.1, below. ECM, including
flowable ECM, is described in Section 4.1.3, below.
In other embodiments, the methods of bioprinting provided herein comprise the
deposition of multiple (e.g., two, three, four, five or more) cell types and flowable ECM on a
surface. In a specific embodiment, the multiple cell types are deposited as part of the same
composition, i.e., the source of the cells is a single composition that comprises the multiple cell
types. In another specific embodiment, the multiple cell types are deposited as part of different
compositions, i.e., the source of the cells are distinct compositions that comprise the multiple cell
types. In another specific embodiment, a portion of the multiple cell types are deposited as part
of one composition (e.g., two or more cell types are in a single composition) and another portion
of the multiple types are deposited as a different composition (e.g., one or more cell types are in
a single composition). Exemplary cell types that can be used in accordance with such methods
are provided in Section 4.1.2, below.
In a specific embodiment, the cells to be deposited and the flowable ECM are
deposited on a surface together (e.g., simultaneously) as part of the same composition. In

another specific embodiment, the cells to be deposited and the flowable ECM are deposited on a
surface together as part of different compositions. In another specific embodiment, the cells to
be deposited and the flowable ECM are deposited on a surface separately (e.g., at different
times).
In certain embodiments, the cells and flowable ECM are deposited with one or more
additional components. In one embodiment, the one or more additional components are
formulated in the same composition as the cells. In another embodiment, the one or more
additional components are formulated in the same composition as the ECM. In another
embodiment, the one or more additional components are formulated in the same composition as
the cells and the ECM (i.e., a single composition comprises the cells, the flowable ECM, and the
one or more additional components). In another embodiment, the one or more additional
components are formulated in a composition that is separate from the compositions comprising
the cells and/or ECM, and is deposited concurrently with, before, or after the deposition of the
cells and/or ECM on a surface. In a specific embodiment, the one or more additional
components promote the survival, differentiation, proliferation, etc. of the cell(s). In another
specific embodiment, the one or more additional components comprise a cross-linker (see
Section 4.1.3.2). In another specific embodiment, the one or more additional components
comprise a hydrogel. In another specific embodiment, the one or more additional components
comprise a synthetic polymer.
Those of skill in the art will recognize that the cells and flowable ECM, as well as any
additional components used in accordance with the methods described herein, may be printed
from separate nozzles of a printer, or through the same nozzle of a printer in a common
composition, depending upon the particular tissue or organ being formed. It also will be
recognized by those of skill in the art that the printing may be simultaneous or sequential, or any
combination thereof and that some of the components (e.g., cells, flowable ECM, or cross-
linkers) may be printed in the form of a first pattern and some of the components may be printed
in the form of a second pattern, and so on. The particular combination and manner of printing
will depend upon the particular tissue or organ being printed.
In certain embodiments, the cells, ECM, and/or any other materials (e.g., synthetic
matrices, e.g., PCL) may be bioprinted in a specified pattern so as to yield a desired result. For
example, bioprinted materials (e.g., cells, ECM, matrices, and other components described

herein) may be bioprinted or otherwise deposited in layers at varying angles so as to generate
specific desirable patterns, such as three-dimensional structures having specific pore sizes. In a
specific embodiment, bioprinted materials (e.g., cells, ECM, matrices, and other components
described herein) are printed or otherwise deposited in a criss-cross fashion so as to generate a
bioprinted structure with pores of desired sizes that appear box-like. In another specific
embodiment, bioprinted materials (e.g., cells, ECM, matrices, and other components described
herein) are printed or otherwise deposited at angles, so as to generate pores of desired sizes that
appear triangular or diamond-like. For example, bioprinted materials (e.g., cells, ECM, matrices,
and other components described herein) can be printed or otherwise deposited at angles of
specific degrees, e.g., 30 degree angles, 45 degree angles, 60 degree angles, in order to generate
desired patterns. In accordance with such methods, structures having desirable qualities, e.g., the
ability to foster cellular growth and proliferation, can be generated. See Example 1, below. In a
specific embodiment, matrices, e.g., synthetic matrices, are bioprinted in specific patterns that
are conducive to supporting the growth and proliferation of cells on said bioprinted matrices. In
specific embodiments, the synthetic matrix is PCL.
4.1.1 CELLS
Any type of cell known in the art can be used in accordance with the methods
described herein, including eukaryotic cells.
The cells used in accordance with the methods described herein may be syngeneic
(i.e., genetically identical or closely related to the cells of the recipient subject, so as to minimize
tissue transplant rejection), allogeneic (i.e., from a non-genetically identical member of the same
species of the recipient subject) or xenogeneic (i.e., from a member of a different species than
the recipient subject). Syngeneic cells include those that are autogeneic (i.e., from the recipient
subject) and isogeneic (i.e., from a genetically identical but different subject, e.g., from an
identical twin). Cells may be obtained from, e.g., a donor (either living or cadaveric) or derived
from an established cell strain or cell line. For example, cells may be harvested from a donor
(e.g., a potential recipient) using standard biopsy techniques known in the art.
In certain embodiments, the cells used in accordance with the methods described
herein are contained within a flowable physiologically-acceptable composition, e.g., water,
buffer solutions (e.g., phosphate buffer solution, citrate buffer solution, etc.), liquid media (e.g.,
0.9N saline solution, Kreb's solution, modified Kreb's solution, Eagle's medium, modified

Eagle's medium (MEM), Dulbecco's Modified Eagle's Medium (DMEM), Hank's Balanced
Salts, etc.), and the like.
In certain embodiments, the cells used in accordance with the methods described
herein may comprise primary cells that have been isolated from a tissue or organ, using one or
more art-known proteases, e.g., collagenase, dispase, trypsin, LIBERASE, or the like. Organ
tissue may be physically dispersed prior to, during, or after treatment of the tissue with a
protease, e.g., by dicing, macerating, filtering, or the like. Cells may be cultured using standard,
art-known cell culture techniques prior to use of the cells in the methods described herein, e.g., in
order to produce homogeneous or substantially homogeneous cell populations, to select for
particular cell types, or the like.
In one embodiment, the cell type(s) used in the methods described herein comprise
stem cells. A non-limiting list of stem cells that can be used in accordance with the methods
described herein includes: embryonic stem cells, embryonic germ cells, induced pluripotent stem
cells, mesenchymal stem cells, bone marrow-derived mesenchymal stem cells (BM-MSCs),
tissue plastic-adherent placental stem cells (PDACs), umbilical cord stem cells, amniotic fluid
stem cells, amnion derived adherent cells (AMDACs), osteogenic placental adherent cells
(OPACs), adipose stem cells, limbal stem cells, dental pulp stem cells, myoblasts, endothelial
progenitor cells, neuronal stem cells, exfoliated teeth derived stem cells, hair follicle stem cells,
dermal stem cells, parthenogenically derived stem cells, reprogrammed stem cells, amnion
derived adherent cells, or side population stem cells.
In a specific embodiment, the methods described herein comprise the use of placental
stem cells (e.g., the placental stem cells described in US 7,468,276 and US 8,057,788). In
another specific embodiment, said placental stem cells are PDACs®. In one embodiment, said
PDACs are CD34–, CD10+, CD105+, and CD200+. In another embodiment, said PDACs are
CD34–, CD10+, CD105+, and CD200+ and additionally are CD45–, CD80–, CD86–, and/or
CD90+.
In another specific embodiment, the methods described herein comprise the use of
AMDACs (e.g., the AMDACs described in international application publication no.
WO10/059828). In one embodiment, said AMDACs are Oct4-. In another embodiment, said
AMDACs are CD49f+. In another embodiment, said AMDACs are Oct4- and CD49f+.

In another specific embodiment, the methods described herein comprise the use of
PDACs and AMDACs.
In another specific embodiment, the methods described herein comprise the use of
BM-MSCs.
In another embodiment, the cell type(s) used in the methods described herein
comprise differentiated cells. In another specific embodiment, the differentiated cell(s) used in
accordance with the methods described herein comprise endothelial cells, epithelial cells, dermal
cells, endodermal cells, mesodermal cells, fibroblasts, osteocytes, chondrocytes, natural killer
cells, dendritic cells, hepatic cells, pancreatic cells, and/or stromal cells. In another specific
embodiment, the cells are insulin-producing cells, e.g., pancreatic cells (e.g., islet cells) or an
insulin-producing cell line, e.g., β-TC-6 cells.
In another specific embodiment, the differentiated cell(s) used in accordance with the
methods described herein comprise salivary gland mucous cells, salivary gland serous cells, von
Ebner's gland cells, mammary gland cells, lacrimal gland cells, ceruminous gland cells, eccrine
sweat gland dark cells, eccrine sweat gland clear cells, apocrine sweat gland cells, gland of Moll
cells, sebaceous gland cells. bowman's gland cells, Brunner's gland cells, seminal vesicle cells,
prostate gland cells, bulbourethral gland cells, Bartholin's gland cells, gland of Littre cells, uterus
endometrium cells, isolated goblet cells, stomach lining mucous cells, gastric gland zymogenic
cells, gastric gland oxyntic cells, pancreatic acinar cells, paneth cells, type II pneumocytes,
and/or clara cells.
In another specific embodiment, the differentiated cell(s) used in accordance with the
methods described herein comprise somatotropes, lactotropes, thyrotropes, gonadotropes,
corticotropes, intermediate pituitary cells, magnocellular neurosecretory cells, gut cells,
respiratory tract cells, thyroid epithelial cells, parafollicular cells, parathyroid gland cells,
parathyroid chief cell, oxyphil cell, adrenal gland cells, chromaffin cells, Leydig cells, theca
interna cells, corpus luteum cells, granulosa lutein cells, theca lutein cells, juxtaglomerular cell,
macula densa cells, peripolar cells, and/or mesangial cells.
In another specific embodiment, the differentiated cell(s) used in accordance with the
methods described herein comprise blood vessel and lymphatic vascular endothelial fenestrated
cells, blood vessel and lymphatic vascular endothelial continuous cells, blood vessel and
lymphatic vascular endothelial splenic cells, synovial cells, serosal cell (lining peritoneal, pleural,

and pericardial cavities), squamous cells, columnar cells, dark cells, vestibular membrane cell
(lining endolymphatic space of ear), stria vascularis basal cells, stria vascularis marginal cell
(lining endolymphatic space of ear), cells of Claudius, cells of Boettcher, choroid plexus cells,
pia-arachnoid squamous cells, pigmented ciliary epithelium cells, nonpigmented ciliary
epithelium cells, corneal endothelial cells, peg cells, respiratory tract ciliated cells, oviduct
ciliated cell, uterine endometrial ciliated cells, rete testis ciliated cells, ductulus efferens ciliated
cells, and/or ciliated ependymal cells.
In another specific embodiment, the differentiated cell(s) used in accordance with the
methods described herein comprise epidermal keratinocytes, epidermal basal cells, keratinocyte
of fingernails and toenails, nail bed basal cells, medullary hair shaft cells, cortical hair shaft cells,
cuticular hair shaft cells, cuticular hair root sheath cells, hair root sheath cells of Huxley's layer,
hair root sheath cells of Henle's layer, external hair root sheath cells, hair matrix cells, surface
epithelial cells of stratified squamous epithelium, basal cell of epithelia, and/or urinary
epithelium cells.
In another specific embodiment, the differentiated cell(s) used in accordance with the
methods described herein comprise auditory inner hair cells of organ of Corti, auditory outer hair
cells of organ of Corti, inner pillar cells of organ of Corti, outer pillar cells of organ of Corti,
inner phalangeal cells of organ of Corti, outer phalangeal cells of organ of Corti, border cells of
organ of Corti, Hensen cells of organ of Corti, vestibular apparatus supporting cells, taste bud
supporting cells, olfactory epithelium supporting cells, Schwann cells, satellite cells, enteric glial
cells, basal cells of olfactory epithelium, cold-sensitive primary sensory neurons, heat-sensitive
primary sensory neurons, Merkel cells of epidermis, olfactory receptor neurons, pain-sensitive
primary sensory neurons, photoreceptor rod cells, photoreceptor blue-sensitive cone cells,
photoreceptor green-sensitive cone cells, photoreceptor red-sensitive cone cells, proprioceptive
primary sensory neurons, touch-sensitive primary sensory neurons, type I carotid body cells, type
II carotid body cell (blood pH sensor), type I hair cell of vestibular apparatus of ear (acceleration
and gravity), type II hair cells of vestibular apparatus of ear, type I taste bud cells, cholinergic
neural cells, adrenergic neural cells, and/or peptidergic neural cells.
In another specific embodiment, the differentiated cell(s) used in accordance with the
methods described herein comprise astrocytes, neurons, oligodendrocytes, spindle neurons,
anterior lens epithelial cells, crystallin-containing lens fiber cells, hepatocytes, adipocytes, white

fat cells, brown fat cells, liver lipocytes, kidney glomerulus parietal cells, kidney glomerulus
podocytes, kidney proximal tubule brush border cells, loop of Henle thin segment cells, kidney
distal tubule cells, kidney collecting duct cells, type I pneumocytes, pancreatic duct cells,
nonstriated duct cells, duct cells, intestinal brush border cells, exocrine gland striated duct cells,
gall bladder epithelial cells, ductulus efferens nonciliated cells, epididymal principal cells, and/or
epididymal basal cells.
In another specific embodiment, the differentiated cell(s) used in accordance with the
methods described herein comprise ameloblast epithelial cells, planum semilunatum epithelial
cells, organ of Corti interdental epithelial cells, loose connective tissue fibroblasts, corneal
keratocytes, tendon fibroblasts, bone marrow reticular tissue fibroblasts, nonepithelial fibroblasts,
pericytes, nucleus pulposus cells, cementoblast/cementocytes, odontoblasts, odontocytes, hyaline
cartilage chondrocytes, fibrocartilage chondrocytes, elastic cartilage chondrocytes, osteoblasts,
osteocytes, osteoclasts, osteoprogenitor cells, hyalocytes, stellate cells (ear), hepatic stellate cells
(Ito cells), pancreatic stelle cells, red skeletal muscle cells, white skeletal muscle cells,
intermediate skeletal muscle cells, nuclear bag cells of muscle spindle, nuclear chain cells of
muscle spindle, satellite cells, ordinary heart muscle cells, nodal heart muscle cells, Purkinje
fiber cells, mooth muscle cells, myoepithelial cells of iris, and/or myoepithelial cells of exocrine
glands.
In another specific embodiment, the differentiated cell(s) used in accordance with the
methods described herein comprise reticulocytes, megakaryocytes, monocytes, connective tissue
macrophages. epidermal Langerhans cells, dendritic cells, microglial cells, neutrophils,
eosinophils, basophils, mast cell, helper T cells, suppressor T cells, cytotoxic T cell, natural
Killer T cells, B cells, natural killer cells, melanocytes, retinal pigmented epithelial cells,
oogonia/oocytes, spermatids, spermatocytes, spermatogonium cells, spermatozoa, ovarian
follicle cells, Sertoli cells, thymus epithelial cell, and/or interstitial kidney cells.
The cells used in accordance with the methods described herein can be formulated in
compositions. In certain embodiments, the cells used in accordance with the methods described
herein are formulated in compositions that comprise only a single cell type, i.e., the population of
cells in the composition is homogeneous. In other embodiments, the cells used in accordance
with the methods described herein are formulated in compositions that comprise more than one
cell type, i.e., the population of cells in the composition is heterogeneous.

In certain embodiments, the cells used in accordance with the methods described
herein are formulated in compositions that additionally comprise flowable ECM (see Section
4.1.3). Alternatively, said flowable ECM may be deposited as part of a separate composition in
accordance with the methods described herein concurrently with, before, or after the deposition
of said cells. In certain embodiments, the cells used in accordance with the methods described
herein are formulated in compositions that additionally comprise one or more synthetic
monomers or polymers. Alternatively, said synthetic monomers or polymers may be deposited
as part of a separate composition in accordance with the methods described herein concurrently
with, before, or after the deposition of said cells. In certain embodiments, the cells used in
accordance with the methods described herein are formulated in compositions that additionally
comprise flowable ECM and one or more synthetic monomers or polymers. In certain
embodiments, the cells used in accordance with the methods described herein are formulated in
compositions that additionally comprise a cross-linking agent. Alternatively, said cross-linking
agent may be deposited as part of a separate composition in accordance with the methods
described herein concurrently with, before, or after the deposition of said cells.
In certain embodiments, the cells used in accordance with the methods described
herein are formulated in compositions that additionally comprise one or more additional
components, e.g., components that promote the survival, differentiation, proliferation, etc. of the
cell(s). Such components may include, without limitation, nutri[?]nts, [?]alts, [?]ugars, su[?]vival
factors, and growth factors. Exemplary growth factors that may be used in accordance with the
methods described herein include, without limitation, insulin-like growth factor (e.g., IGF-1),
transforming growth factor-beta (TGF-beta), bone-morphogenetic protein, fibroblast growth
factor, platelet derived growth factor (PDGF), vascular endothelial growth factor (VEGF),
connective tissue growth factor (CTGF), basic fibroblast growth factor (bFGF), epidermal
growth factor, fibroblast growth factor (FGF) (numbers 1, 2 and 3), osteopontin, bone
morphogenetic protein-2, growth hormones such as somatotropin, cellular attractants and
attachment agents, etc., and mixtures thereof. Alternatively, said one or more additional
components that promote the survival, differentiation, proliferation, etc. of the cell(s) may be
deposited as part of a separate composition in accordance with the methods described herein
concurrently with, before, or after the deposition of said cells.

In certain embodiments, the cells used in accordance with the methods described
herein are formulated in compositions that additionally comprise a polymerizable monomer(s).
Alternatively, said polymerizable monomer may be deposited as part of a separate composition
in accordance with the methods described herein concurrently with, before, or after the
deposition of said cells. In such embodiments, for example, a polymerization catalyst may be
added immediately prior to bioprinting, such that once the cells are printed, the monomer
polymerizes, forming a gel that traps and/or physically supports the cells. For example, the
composition comprising the cells can comprise acrylamide monomers, whereupon TEMED and
Ammonium persulfate, or riboflavin, are added to the composition immediately prior to
bioprinting. Upon deposition of the cells in the composition onto a surface, the acrylamide
polymerizes, sequestering and supporting the cells.
In certain embodiments, the cells used in accordance with the methods described
herein are formulated in compositions that additionally comprise adhesives. In a specific
embodiment, the cells used in accordance with the methods described herein are formulated in
compositions that additionally comprise soft tissue adhesives including, without limitation,
cyanoacrylate esters, fibrin sealant, and/or gelatin-resorcinol-formaldehyde glues. In another
specific embodiment, the cells used in accordance with the methods described herein are
formulated in compositions that additionally comprise arginine-glycine-aspartic acid (RGD)
ligands, extracellular proteins, and/or extracellular protein analogs.
In certain embodiments, the cells used in accordance with the methods described
herein are formulated in compositions such that the cells can be deposited on a surface as single
cells (i.e., the cells are deposited one cell at a time).
In certain embodiments, the cells used in accordance with the methods described
herein are formulated in compositions such that the cells can be deposited on a surface as
aggregates that comprise multiple cells. Such aggregates may comprise cells of single type, or
may comprise multiple cell types, e.g., two, three, four, five or more cell types.
In certain embodiments, the cells used in accordance with the methods described
herein are formulated in compositions such that the cells form a tissue as part of the composition,
wherein said tissue can be deposited on a surface using the methods described herein. Such
tissues may comprise cells of single type, or may comprise multiple cell types, e.g., two, three,
four, five or more cell types.

In certain embodiments, the cells used in accordance with the methods described
herein are deposited onto a surface as individual droplets of cells and/or compositions having
small volumes, e.g., from 0.5 to 500 picoliters per droplet. In various embodiments, the volume
of cells, or composition comprising the cells, is about 0.5, 1, 2, 3, 4, 5, 6, 7, 8, 9, 10, 15, 20, 25,
20, 35, 40, 45, 50, 55, 60, 65, 70, 75, 80, 85, 90, 95 or 100 picoliters, or between about 1 to 90
picoliters, about 5 to 85 picoliters, about 10 to 75 picoliters, about 15 to 70 picoliters, about 20 to
65 picoliters, or about 25 to about 60 picoliters.
4.1.2	TISSUES AND ORGANS
Provided herein are tissues and organs engineered/generated using one or more of the
methods provided herein.
Any type of tissue known in the art can be generated using methods described herein.
In certain embodiments, the tissue generated in accordance with the methods described herein
comprises a single cell type. In other embodiments, the tissue generated in accordance with the
methods described herein comprises multiple cell types. In certain embodiments, the tissue
generated in accordance with the methods described herein comprises more than one type of
tissue.
In certain embodiments, the methods described herein comprise deposition of cells on
a surface, wherein said surface comprises tissue from a subject, e.g., the tissue is from a donor,
from the recipient subject, from a cadaver, or from another source. In certain embodiments, the
methods described herein comprise deposition of cells on a surface, wherein said surface
comprises tissue that is not from a subject, e.g., the tissue has been synthesized.
In a specific embodiment, the tissue generated in accordance with the methods
described herein is connective tissue.
In another specific embodiment, the tissue generated in accordance with the methods
described herein is muscle tissue. The muscle tissue generated in accordance with the methods
described herein can comprise visceral (smooth) muscle tissue, skeletal muscle tissue, or cardiac
muscle tissue.
In another specific embodiment, the tissue generated in accordance with the methods
described herein is neural tissue. The neural tissue generated in accordance with the methods
described herein can comprise central nervous system tissue (e.g., brain tissue or spinal cord
tissue) or peripheral nervous system tissue (e.g., cranial nerves and spinal nerves).

In another specific embodiment, the tissue generated in accordance with the methods
described herein is epithelial tissue, including endothelium.
In certain embodiments, the tissues generated in accordance with the methods
described herein can be used to engineer an organ. In certain embodiments, the tissues generated
in accordance with the methods described herein can be used to engineer a portion of an organ.
The tissues and organs engineered in accordance with the methods described herein
can be associated with any of the known mammalian organ systems, i.e., the digestive system,
circulatory system, endocrine system, excretory system, immune system, integumentary system,
muscular system, nervous system, reproductive system, respiratory system, and/or skeletal
system. Exemplary organs that can be generated or formed in accordance with the methods
described herein include, without limitation, lungs, liver, heart, brain, kidney, skin, bone,
stomach, pancreas, bladder, gall bladder, small intestine, large intestine, prostate, testes, ovaries,
spinal cord, pharynx, larynx, trachea, bronchi, diaphragm, ureter, urethra, esophagus, colon,
thymus, and spleen. In a specific embodiment, a pancreas is generated or formed in accordance
with the methods described herein.
In a specific embodiment, the methods described herein are used to engineer bone. In
another specific embodiment, the methods described herein are used to engineer skin. In another
specific embodiment, the methods described herein are used to engineer lung tissue, or a lung or
portion thereof. In another specific embodiment, the methods described herein are used to
engineer liver tissue, or a liver or portion thereof. In another specific embodiment, the methods
described herein are used to engineer neural tissue, or a nerve or portion thereof.
In certain embodiments, a tissue or organ generated in accordance with the methods
described herein may additionally comprise components beneficial to the function of said tissue
or organ. In certain embodiments, a tissue or organ generated in accordance with the methods
described herein may additionally comprise a nerve guidance conduit, i.e. an artificial means of
guiding axonal regrowth. In a specific embodiment, the nerve guidance conduit is made of a
polyanhydride, e.g., poly(o-carboxyphenoxy)-p-xylene) or poly(lactide-anhydride). In certain
embodiments, the nerve guidance conduit can be deposited simultaneously with the printing of
the tissue or organ, i.e., the nerve guidance conduit is printed along with the tissue or organ. In
other embodiments, the nerve guidance conduit may be prepared prior to the printing of said
tissue or organ and placed (e.g., manually placed) into the tissue or organ as it is being printed.

In other embodiments, the nerve guidance conduit may be prepared prior to the printing of said
tissue or organ and placed (e.g., manually placed) into the tissue or organ after it has been
printed.
In certain embodiments, a tissue or organ generated in accordance with the methods
described herein may additionally comprise blood vessels, e.g., blood vessels obtained from a
subject (e.g., a donor, the recipient subject, or a cadaver) or blood vessels engineered using the
methods described herein.
In certain embodiments, the tissues and organs generated in accordance with the
methods described herein are in the shape of the tissue or organ as it would appear in its natural
state, e.g., in the human body. For example, a lung generated in accordance with the methods
described herein may resemble a human lung as it appears in the human body.
In certain embodiments, the tissues and organs generated in accordance with the
methods described herein are not in the shape of the tissue or organ as it would appear in its
natural state, yet function in the same manner or in a similar manner as does the organ. For
example, a lung generated in accordance with the methods described herein may not resemble a
human lung as it appears in the human body, but may retain some or all of the functions of the
human lung. In such embodiments, the tissues and organs generated in accordance with the
methods described herein can be of various shapes including, without limitation, a sphere, a
cylinder, rod-like, or cuboidal (i.e., cubes).
4.1.3	EXTRACELLULAR MATRIX (ECM)
The methods described herein comprise the deposition of cells (e.g., compositions
comprising single cells and/or compositions comprising multiple cells) and extracellular matrix
(ECM), including flowable ECM, on a surface. The ECM can be derived from any known
source of ECM, and can be made flowable using any method known in the art. In specific
embodiments, the ECM comprises flowable ECM. The ECM can be made flowable using, e.g.,
the methods described in Section 4.1.3.1, below. In certain embodiments, the ECM can be cross-
linked using, e.g., using the methods described in Section 4.1.3.2, below.
The ECM (e.g., a flowable ECM) used in accordance with the methods described
herein can be formulated as part of a composition for use in accordance with the methods
provided herein.

In certain embodiments, the ECM used in accordance with the methods described
herein comprises mammalian ECM, plant ECM, molluscan ECM, and/or piscine ECM.
In a specific embodiment, the ECM used in accordance with the methods described
herein comprises mammalian ECM.  In another specific embodiment, the ECM used in
accordance with the methods described herein comprises mammalian ECM, wherein said
mammalian ECM is derived from a placenta (e.g., a human placenta).  In another specific
embodiment, said placental-derived ECM comprises telopeptide collagen.
In another specific embodiment, said placental-derived ECM comprises base-treated
and/or detergent treated Type I telopeptide placental collagen that has not been chemically
modified or contacted with a protease, wherein said ECM comprises less than 5% fibronectin or
less than 5% laminin by weight; between 25% and 92% Type I collagen by weight; and 2% to
50% Type III collagen or 2% to 50% type IV collagen by weight.
In another specific embodiment, said placental-derived ECM comprises base-treated,
detergent treated Type I telopeptide placental collagen that has not been chemically modified or
contacted with a protease, wherein said ECM comprises less than 1% fibronectin or less than 1%
laminin by weight; between 74% and 92% Type I collagen by weight; and 4% to 6% Type III
collagen or 2% to 15% type IV collagen by weight.
Placental ECM, e.g., ECM comprising placental telopeptide collagen, used in
accordance with the methods described herein, may be prepared using methods known in the art,
or may be prepared as follows.  First, placental tissue (either whole placenta or part thereof) is
obtained by standard methods, e.g., collection as soon as practical after Caesarian section or
normal birth, e.g., aseptically.  The placental tissue can be from any part of the placenta
including the amnion, whether soluble or insoluble or both, the chorion, the umbilical cord or
from the entire placenta.  In certain embodiments, the collagen composition is prepared from
whole human placenta without the umbilical cord.  The placenta may be stored at room
temperature, or at a temperature of about 2° C to 8° C, until further treatment.  The placenta is
preferably exsanguinated, i.e., completely drained of the placental and cord blood remaining
after birth.  The expectant mother, in certain embodiments, is screened prior to the time of birth,
for, e.g., HIV, HBV, HCV, HTLV, syphilis, CMV, and other viral pathogens known to
contaminate placental tissue.

The placental tissue may be decellularized prior to production of the ECM. The
placental tissue can be decellularized according to any technique known to those of skill in the
art such as those described in detail in U.S. Patent Application Publication Nos. 20040048796
and 20030187515, the contents of which are hereby incorporated by reference in their entireties.
The placental tissue may be subjected to an osmotic shock. The osmotic shock can be
in addition to any clarification step or it can be the sole clarification step according to the
judgment of one of skill in the art. The osmotic shock can be carried out in any osmotic shock
conditions known to those of skill in the art. Such conditions include incubating the tissue in
solutions of high osmotic potential, or of low osmotic potential or of alternating high and low
osmotic potential. The high osmotic potential solution can be any high osmotic potential
solution known to those of skill in the art such as a solution comprising one or more of NaCl
(e.g., 0.2-1.0 M or 0.2-2.0 M), KCl (e.g., 0.2-1.0 or 0.2 to 2.0 M), ammonium sulfate, a
monosaccharide, a disaccharide (e.g., 20% sucrose), a hydrophilic polymer (e.g., polyethylene
glycol), glycerol, etc. In certain embodiments, the high osmotic potential solution is a sodium
chloride solution, e.g., at least 0.25 M, 0.5M, 0.75M, 11.0M, 1.25M, 1.5M, 1.75M, 2M, or 2.5M
NaCl. In some embodiments, the sodium chloride solution is about 0.25-5M, about 0.5-4M,
about 0.75-3M, or about 1.0-2.0M NaCl. The low osmotic potential solution can be any low
osmotic potential solution known to those of skill in the art, such as water, for example water
deionized according to any method known to those of skill. In some embodiments, the osmotic
shock solution comprises water with an osmotic shock potential less than that of 50 mM NaCl.
In certain embodiments, the osmotic shock is in a sodium chloride solution followed by a water
solution. In certain embodiments, one or two NaCl solution treatments are followed by a water
wash.
The composition resulting from the osmotic shock may then, in certain embodiments,
be incubated with a detergent. The detergent can be any detergent known to those of skill in the
art to be capable of disrupting cellular or subcellular membranes, e.g., an ionic detergent, a
nonionic detergent, deoxycholate, sodium dodecylsulfate, Triton X 100, TWEEN, or the like.
Detergent treatment can be carried out at about 0° C to about 30° C, about 5° C to about 25° C,
about 5° C to about 20 ° C, about 5° C to about 15° C, about 0° C, about 5° C, about 10° C, about
15° C, about 20° C, about 25° C., or about 30° C. Detergent treatment can be carried out for, e.g.,
about 1-24 hours, about 2-20 hours, about 5-15 hours, about 8-12 hours, or about 2-5 hours.

The composition resulting from the detergent treatment may then, in certain
embodiments, be incubated under basic conditions. Particular bases for the basic treatment
include biocompatible bases, volatile bases, or any organic or inorganic bases at a concentration
of, for example, 0.2-1.0M. In certain embodiments, the base is selected from the group
consisting of NH4OH, KOH and NaOH, e.g., 0.1M NaOH, 0.25M NaOH, 0.5M NaOH, or 1M
NaOH. The base treatment can be carried out at, e.g., 0° C to 30° C., 5° C to 25° C., 5° C to 20°
C., 5° C to 15° C, about 0° C., about 5° C., about 10° C., about 15° C., about 20° C., about 25° C.,
or about 30° C, for, e.g., about 1-24 hours, about 2-20 hours, about 5-15 hours, about 8-12 hours,
or about 2-5 hours.
The ECM can be produced without treatment by a base; omission of a base treatment
step typically results in an ECM composition comprising relatively higher amounts of elastin,
fibronectin and/or laminin than the ECM composition produced with inclusion of the basic
treatment.
Typically, the process described above for human placental tissue results in
production of placental ECM comprising base-treated and/or detergent treated Type I telopeptide
placental collagen that has not been chemically modified or contacted with a protease, wherein
said ECM comprises less than 5% fibronectin or less than 5% laminin by weight; between 25%
and 92% Type I collagen by weight; between 2% and 50% Type III collagen; between 2% and
50% type IV collagen by weight; and/or less than 40% elastin by weight. In a more specific
embodiment, the process results in production of base-treated, detergent treated Type I
telopeptide placental collagen, wherein said collagen has not been chemically modified or
contacted with a protease, and wherein said composition comprises less than 1% fibronectin by
weight; less than 1% laminin by weight; between 74% and 92% Type I collagen by weight;
between 4% and 6% Type III collagen by weight; between 2% and 15% type IV collagen by
weight; and/or less than 12% elastin by weight.
In certain embodiments, compositions provided herein that comprise flowable ECM
may additionally comprise other components. In certain embodiments, the compositions
provided herein that comprise flowable ECM additionally comprise one or more cell types, e.g.,
one or more of the cell types detailed in Section 4.1.1, above. Alternatively, said cells may be
deposited as part of a separate composition in accordance with the methods described herein
concurrently with, before, or after the deposition of said ECM.

In certain embodiments, the compositions provided herein that comprise flowable
ECM additionally comprise a hydrogel (e.g., a thermosensitive hydrogel and/or a photosensitive
hydrogel). Alternatively, a hydrogel may be deposited as part of a separate composition in
accordance with the methods described herein concurrently with, before, or after the deposition
of said ECM.
In certain embodiments, the compositions provided herein that comprise flowable
ECM additionally comprise one or more cell types, e.g., one or more of the cell types detailed in
Section 4.1.1, above, and a hydrogel. In a specific embodiment, the compositions provided
herein that comprise flowable ECM and a hydrogel (e.g., a thermosensitive hydrogel and/or a
photosensitive hydrogel) are formulated such that the ratio of ECM:hydrogel ranges from about
10:1 to about 1:10 by weight.
Exemplary hydrogels may comprise include organic polymers (natural or synthetic)
that may be cross-linked via covalent, ionic, or hydrogen bonds to create a three-dimensional
open-lattice structure that entraps water molecules to form a gel. Suitable hydrogels for such
compositions include self-assembling peptides, such as RAD16. Hydrogel-forming materials
include polysaccharides such as alginate and salts thereof, peptides, polyphosphazines, and
polyacrylates, which are crosslinked ionically, or block polymers such as polyethylene oxide-
polypropylene glycol block copolymers which are crosslinked by temperature or pH,
respectively. In some embodiments, the hydrogel or matrix may be biodegradable.
In certain embodiments, the compositions provided herein that comprise flowable
ECM additionally comprise a synthetic polymer. In a specific embodiment, the synthetic
polymer comprises polyacrylamide, polyvinylidine chloride, poly(o-carboxyphenoxy)-p-xylene)
(poly(o-CPX)), poly(lactide-anhydride) (PLAA), n-isopropyl acrylamide, pent erythritol
diacrylate, polymethyl acrylate, carboxymethylcellulose, and/or poly(lactic-co-glycolic acid)
(PLGA). In another specific embodiment, the synthetic polymer comprises a thermoplastic, e.g.,
polycaprolactone (PCL), polylactic acid, polybutylene terephthalate, polyethylene terephthalate,
polyethylene, polyester, polyvinyl acetate, and/or polyvinyl chloride. Alternatively, one or more
synthetic polymers may be deposited as part of a separate composition in accordance with the
methods described herein concurrently with, before, or after the deposition of said ECM. In a
specific embodiment, the synthetic polymer is PCL.

In certain embodiments, the compositions provided herein that comprise flowable
ECM additionally comprise tenascin C, a human protein known to interact with fibronectin, or a
fragment thereof.  Alternatively, tenascin C may be deposited as part of a separate composition
in accordance with the methods described herein concurrently with, before, or after the
deposition of said ECM.
In certain embodiments, the compositions provided herein that comprise flowable
ECM additionally comprise titanium-aluminum-vanadium (Ti6Al4V).  Alternatively, Ti6Al4V
may be deposited as part of a separate composition in accordance with the methods described
herein concurrently with, before, or after the deposition of said ECM.
In certain embodiments, the ECM in a composition provided herein and/or an
additional component of the composition, such as a synthetic polymer, may be derivatized.
Methods for derivatization of ECM and synthetic polymers are known in the art, and include,
without limitation, derivatization using cell attachment peptides (e.g., a peptide comprising one
or more RGD motifs), derivatization using cell attachment proteins, derivatization using
cytokines (e.g., vascular endothelial growth factor (VEGF), or a bone morphogenetic protein
(BMP)), and derivatization using glycosaminoglycans.
          4.1.3.1 Methods of Generating Flowable ECM
The ECM used in accordance with the methods described herein can be made
flowable using methods known in the art and described herein.
In one embodiment, the ECM used in accordance with the methods described herein
is made flowable by contacting the ECM with an acid or base, e.g., an acidic or basic solution
comprising an amount of said acid or base that is sufficient to solubilize said ECM.  Once the
ECM has been made flowable, if desired, the ECM containing composition can be made neutral,
or brought to a desired pH, using methods known in the art.
In another embodiment, the ECM used in accordance with the methods described
herein is made flowable by contacting the ECM with an enzyme or combination of enzymes,
e.g., a protease, such as trypsin, chymotrypsin, pepsin, papain, and/or elastase.  Once the ECM
has been made flowable, if desired, the enzymes can be inactivated using methods known in the
art.
In another embodiment, the ECM used in accordance with the methods described
herein is made flowable using physical approaches.  In a specific embodiment, the ECM used in

accordance with the methods described herein is made flowable by milling the ECM, i.e.,
grinding the ECM so as to overcome of the interior bonding forces. In another specific
embodiment, the ECM used in accordance with the methods described herein is made flowable
by shearing the ECM, e.g., with a blender or other source. In another specific embodiment, the
ECM used in accordance with the methods described herein is made flowable by cutting the
ECM. In certain embodiments, when ECM is made more flowable by use of physical
approaches, the ECM may be manipulated in a frozen state (e.g., the ECM is freeze-dried or
frozen in liquid nitrogen).
4.1.3.2 Methods of Cross-linking ECM
The ECM used in accordance with the methods described herein can be cross-linked
using methods known in the art and described herein.
In certain embodiments, the ECM is cross-linked before it is applied to a surface, i.e.,
the ECM may be cross-linked before printing. In accordance with such embodiments, a cross-
linker may be included in a composition that comprises the ECM and, if necessary, the
composition comprising the ECM and cross-linker may be treated under conditions that give rise
to the cross-linking of the ECM before the printing of the ECM.
In other embodiments, the ECM is cross-linked after it is applied to a surface, i.e., the
ECM may be cross-linked after printing. In one embodiment, the ECM is cross-linked after it is
applied to a surface by first printing the ECM onto said surface, followed by printing of a cross-
linker to said surface (i.e., the ECM and the cross-linker are printed as separate compositions).
In accordance with this embodiment, if necessary, the ECM can subsequently be cross-linked by
treating the ECM and cross-linker under conditions that give rise to the cross-linking of the
ECM.
In another embodiment, the ECM is cross-linked after it is applied to a surface by
printing a composition comprising both the ECM and a cross-linker onto a surface and, after said
printing, treating the ECM and cross-linker under conditions that give rise to the cross-linking of
the ECM.
In a specific embodiment, the ECM is cross-linked by chemical cross-linking of
hyaluronic acid, an ECM component. Exemplary means of chemical cross-linking hyaluronic
acid include, without limitation, divinylsulfone cross-linking, bis-epoxide cross-linking, benzyl
ester cross-linking, butanediol diglycidyl ether (BDDE) cross-linking, disulfide cross-linking via

thiol modification, haloacetate modification of the HA, dihydrazide modification of the HA,
tyramine modification of the HA, and the use of Huisgen cycloaddition (i.e., "Click Chemistry").
Such methods are known in the art and further described in, e.g., Burdick and Prestwich, 2011,
Adv. Mater. 23:H41-H56.
In another specific embodiment, the ECM is cross-linked by chemical cross-linking
of ECM proteins. Exemplary chemicals capable of cross-linking ECM proteins include, without
limitation, glutaraldehyde, hexamethylene diisocyanate (HDMI), genipin, carbodiimide,
polyethylene glycol, benzoyl peroxide, BioGlue (a glutaraldehyde based cross-linker; Cryolife
Inc.), polyphosphoesters, and hydrolyzable polyrotaxane.
In another specific embodiment, the ECM is cross-linked by photopolymerization of
hyaluronic acid using, e.g., methacrylic anhydride and/or Glycidyl methacrylate (see, e.g., .,
Burdick and Prestwich, 2011, Adv. Mater. 23:H41-H56).
In another specific embodiment, the ECM is cross-linked by the use of enzymes.
Enzymes suitable for cross-linking of ECM include, without limitation, lysyl oxidase (see, e.g.,
Levental et al., 2009, Cell 139:891-906) and tissue type transglutaminases (see, e.g., Griffin et
al., 2002, J. Biochem. 368:377-96).
Those of skill in the art will recognize that the cross-linkers should be selected based
on the intended use of the bioprinted product. For example, when a method described herein is
used to generate a tissue or organ that is to be administered to a subject, care should be taken to
select and use cross-linkers that will be biocompatible, i.e., non-harmful to said subject.
Alternatively, when a method described herein is used to generate a tissue or organ that is not to
be administered to a subject, e.g., a tissue or organ to be used in diagnostic assays, then the
practitioner of the method may not need to use care in selection of the cross-linker.
4.1.4     SURFACES
Any suitable surface can be used as the surface upon which the cells, flowable ECM,
and/or any additional components can be deposited (e.g., printed) so as to yield the tissues and
organs generated in accordance with methods described herein. Such surfaces may be two-
dimensional (e.g., flat, planar surfaces) or may be three-dimensional.
In one embodiment, the surface upon which the cells, flowable ECM, and/or any
additional components are deposited comprises an artificial surface, i.e., a surface that has been
man-made. In a specific embodiment, said artificial surface is a prosthetic. In certain

embodiments, an artificial surface is selected based on its suitability for administration to and/or
transplantation in a subject, e.g., a human subject. For example, an artificial surface known not
to be immunogenic (i.e., a surface that does not elicit a host immune response) may be selected
for use when the tissue or organ to be deposited on the artificial surface is being made with the
intent that it be transplanted in a subject. In certain embodiments, an artificial surface may be
treated so as to render it suitable for administration to and/or transplantation in a subject, e.g., a
human subject.
In one embodiment, the surface upon which the cells, flowable ECM, and/or any
additional components are deposited comprises a plastic surface. Exemplary types of plastic
surfaces onto which said cells, ECM, and/or additional components can be deposited include,
without limitation, polyester, polyethylene terephtalate, polyethylene, polyvinyl chloride,
polyvinylidene chloride, polypropylene, polystyrene, polyamides, polycarbonate, and
polyurethanes.
In one embodiment, the surface upon which the cells, flowable ECM, and/or any
additional components are deposited comprises a metal surface. Exemplary types of plastic
surfaces onto which said cells, ECM, and/or additional components can be deposited include,
without limitation, aluminum, chromium, cobalt, copper, gold, iron, lead, magnesium,
manganese, mercury, nickel, platinum, silver, tin, titanium, tungsten, and zinc.
In certain embodiments, the artificial surfaces upon which the cells, flowable ECM,
and/or any additional components are deposited are engineered so that they form a particular
shape. For example, an artificial surface may be engineered so that is the shape of a bone (e.g.,
an otic bone), and the appropriate cells (e.g., osteocytes, osteoblasts, osteoclasts and other bone-
related cells), flowable ECM, and/or any additional components may be deposited on and/or in
said surface so as to generate a bone that is suitable for transplantation in a subject.
In another embodiment, said surface comprises a tissue or an organ from a subject
(e.g., a human subject) or a tissue or an organ that is derived from cells of a subject. In certain
embodiments, the surface of said tissue or organ from a subject may be decellularized, e.g.,
treated so as to remove cells from all or part of the surface of the tissue or organ. In a specific
embodiment, the subject from which the surface tissue or surface organ is from is the subject that
is the intended recipient of the tissue or organ to be generated in accordance with the methods
described herein. In another specific embodiment, the subject from which the surface tissue or

surface organ is from is not the subject that is the intended recipient of the tissue or organ to be
generated in accordance with the methods described herein (e.g., the subject that provides the
surface tissue or surface organ to be printed on is a donor or cadaveric subject).
In accordance with the methods described herein, cells, flowable ECM, and/or any
additional components may be deposited on (e.g., printed on) any suitable tissue or organ from a
subject.  In a specific embodiment, the tissue that provides the printing surface is connective
tissue (including bone), muscle tissue (including visceral (smooth) muscle tissue, skeletal muscle
tissue, and cardiac muscle tissue), neural tissue (including central nervous system tissue (e.g.,
brain tissue or spinal cord tissue) or peripheral nervous system tissue (e.g., cranial nerves and
spinal nerves)), or epithelial tissue (including endothelium).  In another specific embodiment, the
organ that provides the printing surface is from any of the known mammalian organ systems,
including the digestive system, circulatory system, endocrine system, excretory system, immune
system, integumentary system, muscular system, nervous system, reproductive system,
respiratory system, and/or skeletal system.  In another specific embodiment, the organ that
provides the printing surface is all or part of a lung, liver, heart, brain, kidney, skin, bone,
stomach, pancreas, bladder, gall bladder, small intestine, large intestine, prostate, testes, ovaries,
spinal cord, pharynx, larynx, trachea, bronchi, diaphragm, ureter, urethra, esophagus, colon,
thymus, and spleen.  In another specific embodiment, the organ that provides the printing surface
is a pancreas, or a portion thereof.
In a specific embodiment, the cells, flowable ECM, and/or any additional components
are deposited on (e.g., printed on) a surface that comprises or consists of bone.  Exemplary bones
that can be printed on include long bones, short bones, flat bones, irregular bones, and seismoid
bones.  Specific bones that can be printed on include, without limitation, cranial bones, facial
bones, otic bones, bones of the phalanges, arm bones, leg bones, ribs, bones of the hands and
fingers, bones of the feet and toes, ankle bones, wrist bones, chest bones (e.g., the sternum), and
the like.
In certain embodiments, the surfaces described herein that serve as scaffolds for the
deposition (e.g., deposition by bioprinting or by other means) of cells, flowable ECM, and/or any
additional components are surfaces that have not been bioprinted.  In certain embodiments, the
surfaces described herein that serve as scaffolds for the deposition (e.g., deposition by
bioprinting or by other means) of cells, flowable ECM, and/or any additional components are

surfaces that have been bioprinted, e.g., bioprinted in accordance with the methods described
herein. In a specific embodiment, the bioprinted surface comprises a synthetic material. In a
specific embodiment, the synthetic material is PCL.

4.2 COMPOSITIONS

Provided herein are compositions that can be used in accordance with the methods
described herein. In one embodiment, provided herein are compositions comprising cells (e.g.,
the cells described in Section 4.1.1, above) that are suitable for use in accordance with the
methods described herein. In another embodiment, provided herein are compositions comprising
flowable ECM (e.g., the flowable ECM described in Section 4.1.3, above) that is suitable for use
in accordance with the methods described herein. In another embodiment, provided are
compositions comprising one or more cross-linkers (e.g., the cross-linkers described in Section
4.1.3.2, above) suitable for use in accordance with the methods described herein. .
In one embodiment, provided herein is a composition comprising cells (e.g., the cells
described in Section 4.1.1, above) and flowable ECM (e.g., the flowable ECM described in
Section 4.1.3, above). In a specific embodiment, the cells comprise stem cells, e.g., bone
marrow-derived mesenchymal stem cells (BM-MSCs), tissue plastic-adherent placental stem
cells (PDACs), and/or amnion derived adherent cells (AMDACs). In another specific
embodiment, the flowable ECM is derived from placenta (e.g., human placenta).
In another embodiment, provided herein is a composition comprising flowable ECM
(e.g., the flowable ECM described in Section 4.1.3, above) and one or more cross-linkers (e.g.,
the cross-linkers described in Section 4.1.3.2, above).
In another embodiment, provided herein is a composition comprising cells (e.g., the
cells described in Section 4.1.1, above) and one or more cross-linkers (e.g., the cross-linkers
described in Section 4.1.3.2, above).
In another embodiment, provided herein is a composition comprising cells (e.g., the
cells described in Section 4.1.1, above), flowable ECM (e.g., the flowable ECM described in
Section 4.1.3, above), and one or more cross-linkers (e.g., the cross-linkers described in Section
4.1.3.2, above).
In a specific embodiment, a composition provided herein comprises stem cells and
flowable ECM, wherein said stem cells are PDACs and wherein said flowable ECM is derived

from placenta.  In another specific embodiment, a composition provided herein comprises stem
cells and a cross-linker, wherein said stem cells are PDACs.  In another specific embodiment, a
composition provided herein comprises stem cells, flowable ECM, and a cross-linker, wherein
said stem cells are PDACs and wherein said flowable ECM is derived from placenta.
In another specific embodiment, a composition provided herein comprises stem cells
and flowable ECM, wherein said stem cells are AMDACs and wherein said flowable ECM is
derived from placenta.  In another specific embodiment, a composition provided herein
comprises stem cells and a cross-linker, wherein said stem cells are AMDACs.  In another
specific embodiment, a composition provided herein comprises stem cells, flowable ECM, and a
cross-linker, wherein said stem cells are AMDACs and wherein said flowable ECM is derived
from placenta.
In another specific embodiment, a composition provided herein comprises stem cells
and flowable ECM, wherein said stem cells are BM-MSCs and wherein said flowable ECM is
derived from placenta.  In another specific embodiment, a composition provided herein
comprises stem cells and a cross-linker, wherein said stem cells are BM-MSCs.  In another
specific embodiment, a composition provided herein comprises stem cells, flowable ECM, and a
cross-linker, wherein said stem cells are BM-MSCs and wherein said flowable ECM is derived
from placenta.
The compositions provided herein, in addition to comprising cells (e.g., the cells
described in Section 4.1.1, above) and/or flowable ECM (e.g., the flowable ECM described in
Section 4.1.3, above) and/or one or more cross-linkers (e.g., the cross-linkers described in
Section 4.1.3.2, above) may additionally comprise other components.  In certain embodiments,
the compositions provided herein additionally comprise a hydrogel (e.g., a thermosensitive
hydrogel and/or a photosensitive hydrogel.  Alternatively, a hydrogel may be formulated in a
composition separate from the cell and ECM comprising compositions provided herein.  In
certain embodiments, the compositions provided herein additionally comprise a synthetic
polymer, such as polyacrylamide, polyvinylidine chloride, poly(o-carboxyphenoxy)-p-xylene)
(poly(o-CPX)),  poly(lactide-anhydride) (PLAA), n-isopropyl acrylamide, pent erythritol
diacrylate, polymethyl acrylate, carboxymethylcellulose, poly(lactic-co-glycolic acid) (PLGA),
and/or a thermoplastic (e.g., polycaprolactone, polylactic acid, polybutylene terephthalate,
polyethylene terephthalate, polyethylene, polyester, polyvinyl acetate, and/or polyvinyl

chloride). Alternatively, a synthetic polymer may be formulated in a composition separate from
the cell and ECM comprising compositions provided herein. In certain embodiments, the
compositions provided herein additionally comprise tenascin C or a fragment thereof.
Alternatively, tenascin C or a fragment thereof may be formulated in a composition separate
from the cell and ECM comprising compositions provided herein. In certain embodiments, the
compositions provided herein that additionally comprise titanium-aluminum-vanadium
(Ti6Al4V). Alternatively, Ti6Al4V may be formulated in a composition separate from the cell
and ECM comprising compositions provided herein. In certain embodiments, the compositions
provided herein additionally comprise a drug (e.g., a small molecule drug). Alternatively, a drug
may be formulated in a composition separate from the cell and ECM comprising compositions
provided herein. In certain embodiments, the compositions provided herein additionally
comprise an antibody (e.g., a therapeutic antibody). Alternatively, an antibody may be
formulated in a composition separate from the cell and ECM comprising compositions provided
herein.
In certain embodiments, the compositions provided herein additionally comprise one
or more additional components that promote the survival, differentiation, proliferation, etc. of the
cell(s) used in the compositions. Such components may include, without limitation, nutrients,
salts, sugars, survival factors, and growth factors. Exemplary growth factors that may be used in
accordance with the methods described herein include, without limitation, insulin-like growth
factor (e.g., IGF-1), transforming growth factor-beta (TGF-beta), bone-morphogenetic protein,
fibroblast growth factor, platelet derived growth factor (PDGF), vascular endothelial growth
factor (VEGF), connective tissue growth factor (CTGF), basic fibroblast growth factor (bFGF),
epidermal growth factor, fibroblast growth factor (FGF) (numbers 1, 2 and 3), osteopontin, bone
morphogenetic protein-2, growth hormones such as somatotropin, cellular attractants and
attachment agents, etc., and mixtures thereof. Alternatively, one or more additional components
that promote the survival, differentiation, proliferation, etc. of the cell(s) may be formulated in a
composition separate from the cell and ECM comprising compositions provided herein.

4.3 USES

The tissues and organs generated in accordance with the methods described herein
can be used for any suitable purpose. In a specific embodiment, the tissues and organs generated

in accordance with the methods described herein are used for therapeutic purposes, e.g., the
tissues and/or organs are used in transplants. In another specific embodiment, the organs
generated in accordance with the methods described herein are used for experimental purposes,
e.g., to assess the effect of one or more compounds and/or surgical procedures on said tissue or
organ.
4.3.1 Therapeutic Uses
In certain embodiments, the tissues and/or organs generated in accordance with the
methods described herein are transplanted to a subject in need of such transplantation.
Exemplary tissues and organs that can be transplanted in an individual are described in Section
4.1.2. Methods of transplantation, including grafting (e.g., skin grafting) and surgical
transplantation procedures are well-known to those of skill in the art.
In certain embodiments, the cells and/or ECM from which the transplanted tissue
and/or organ is derived are from the transplant recipient. In other embodiments, the cells and/or
ECM from which the transplanted tissue and/or organ is derived are not from the transplant
recipient, but are from another subject, e.g., a donor, a cadaver, etc.
In certain embodiments, the cells from which the transplanted tissue and/or organ is
derived are from the transplant recipient, and the ECM from which the transplanted tissue and/or
organ is derived is not from the transplant recipient, but is from another source. In a specific
embodiment, the ECM from which the transplanted tissue and/or organ is derived is from a
placenta (e.g., a human placenta).
In certain embodiments, the ECM from which the transplanted tissue and/or organ is
derived is from the transplant recipient, and the cells from which the transplanted tissue and/or
organ is derived are not from the transplant recipient, but are from another source.
In a specific embodiment, the methods described herein are used to generate skin that
is suitable for transplantation, and said skin is transplanted (i.e., grafted) in a subject in need of
such transplantation (e.g., a burn victim). In a specific embodiment, said subject is human.
In another specific embodiment, the methods described herein are used to generate a
bone that is suitable for transplantation, and said bone is transplanted (e.g., surgically
transplanted) in a subject in need of such transplantation (e.g., someone suffering from
osteoporosis or bone cancer). In a specific embodiment, said subject is human.

In another specific embodiment, the methods described herein are used to generate a
liver, or portion thereof, that is suitable for transplantation, and said liver or portion thereof is
transplanted (e.g., surgically transplanted) in a subject in need of such transplantation (e.g.,
someone suffering from cirrhosis of the liver, hepatitis, or liver cancer).  In a specific
embodiment, said subject is human.
In another specific embodiment, the methods described herein are used to generate a
lung, or portion thereof, that is suitable for transplantation, and said lung or portion thereof is
transplanted (e.g., surgically transplanted) in a subject in need of such transplantation (e.g.,
someone suffering from lung cancer).  In a specific embodiment, said subject is human.
In another specific embodiment, the methods described herein are used to generate a
neural tissue that is suitable for transplantation (e.g., brain tissue or spinal cord tissue), and said
neural tissue is transplanted (e.g., surgically transplanted) in a subject in need of such
transplantation.  In a specific embodiment, said subject has been diagnosed with a neural disease
(i.e., a disease of the central or peripheral nervous system).  In another specific embodiment, said
subject has suffered trauma that has damaged the central or peripheral nervous system of the
subject, e.g., the subject has suffered a traumatic brain injury (TBI) or spinal cord injury (SCI).
In another specific embodiment, said subject is human.
In another specific embodiment, the methods described herein are used to generate a
circulatory system tissue that is suitable for transplantation (e.g., heart tissue, arteries, or veins),
and said circulatory system tissue is transplanted (e.g., surgically transplanted) in a subject in
need of such transplantation.  In a specific embodiment, said subject is human.
4.3.1.1 Patient Populations
The tissues and/or organs generated in accordance with the methods described herein
can be used to benefit various patient populations.  In one embodiment, the tissues and/or organs
generated in accordance with the methods described herein are used in subjects requiring
transplantation of a tissue and/or organ.
In a specific embodiment, a tissue(s) and/or organ(s) generated in accordance with the
methods described herein is transplanted in a subject that has been diagnosed with cancer, i.e., to
replace all or part of one or more of the organs/tissues of said subject that have been affected by
the cancer.  In a specific embodiment, a tissue(s) and/or organ(s) generated in accordance with
the methods described herein is transplanted in a subject that has been diagnosed with a bone or

connective tissue sarcoma, brain cancer, breast cancer, ovarian cancer, kidney cancer, pancreatic
cancer, esophageal cancer, stomach cancer, liver cancer, lung cancer (e.g., small cell lung cancer
(SCLC), non-small cell lung cancer (NSCLC), throat cancer, and mesothelioma), and/or prostate
cancer.
In another specific embodiment, a lung tissue(s) and/or organ(s) generated in
accordance with the methods described herein is transplanted in a subject that has been
diagnosed with a respiratory disease, e.g., the subject has been diagnosed with asthma, chronic
obstructive pulmonary disorder (COPD), emphysema, pneumonia, tuberculosis, lung cancer
and/or cystic fibrosis.
In another specific embodiment, a liver tissue(s) and/or organ(s) generated in
accordance with the methods described herein is transplanted in a subject that has been
diagnosed with a liver disease, e.g., the subject has been diagnosed with hepatitis (e.g., Hepatitis
A, B, or C), liver cancer, hemochromatosis, or cirrhosis of the liver.
In another specific embodiment, a bone tissue(s) and/or organ(s) generated in
accordance with the methods described herein is transplanted in a subject that has been
diagnosed with a bone disease, e.g., the subject has been diagnosed with bone cancer (e.g.,
osteosarcoma), osteonecrosis, metabolic bone disease, Fibrodysplasia ossificans progressive, or
osteoporosis.
In another specific embodiment, a neural tissue(s) and/or organ(s) generated in
accordance with the methods described herein is transplanted in a subject that has been
diagnosed with a neural disease (i.e., a disease of the central or peripheral nervous system), e.g.,
the subject has been diagnosed with brain cancer, encephalitis, meningitis, Alzheimer's disease,
Parkinson's disease, stroke, or multiple sclerosis.
In another specific embodiment, an epidermal (e.g., skin) tissue(s) generated in
accordance with the methods described herein is transplanted in a subject that has been
diagnosed with a skin disease (i.e., a disease that affects the skin), e.g., the subject has been
diagnosed with skin cancer, eczema, acne, psoriasis, shingles, keratosis; or the subject has
scarring.
In another specific embodiment, a neural tissue(s) and/or organ(s) generated in
accordance with the methods described herein is transplanted in a subject that has undergone

trauma that has damaged the central or peripheral nervous system of the subject, e.g., the subject
has suffered a traumatic brain injury (TBI) or spinal cord injury (SCI).
In another specific embodiment, a circulatory system tissue(s) and/or organ(s)
generated in accordance with the methods described herein is transplanted in a subject that has
been diagnosed with a disease of the circulatory system, e.g., the subject has been diagnosed
with coronary heart disease, cardiomyopathy (e.g., intrinsic or extrinsic cardiomyopathy), heart
attack, stroke, inflammatory heart disease, hypertensive heart disease, or valvular heart disease.
In some embodiments, a subject to which a tissue or organ generated in accordance
with the methods described herein is transplanted is an animal. In certain embodiments, the
animal is a bird. In certain embodiments, the animal is a canine. In certain embodiments, the
animal is a feline. In certain embodiments, the animal is a horse. In certain embodiments, the
animal is a cow. In certain embodiments, the animal is a mammal, e.g., a horse, swine, mouse,
or primate, preferably a human. In a specific embodiment, a subject to which a tissue or organ
generated in accordance with the methods described herein is transplanted is a human.
In certain embodiments, a subject to which a tissue or organ generated in accordance
with the methods described herein is transplanted is a human adult. In certain embodiments, a
subject to which a tissue or organ generated in accordance with the methods described herein is
transplanted is a human infant. In certain embodiments, a subject to which a tissue or organ
generated in accordance with the methods described herein is transplanted is a human child.
4.3.2     Experimental Uses
In certain embodiments, the tissues and/or organs generated in accordance with the
methods described herein are used for experimental purposes.
In a specific embodiment, the tissues and/or organs generated in accordance with the
methods described herein are used for screening the effect of drugs on said tissues and/or organs.
In accordance with such methods, a tissue or organ generated in accordance with the methods
described herein can be exposed to a given drug (e.g., a drug to be assessed) and to a control
(e.g., a composition that does not comprise the drug), and the effect of the drug on the tissue or
organ can be assessed using methods known to those of skill in the art (e.g., by assessing toxicity
of the drug as compared to the control; efficacy of the drug to cause a certain result as compared
to the control, etc.).

In certain embodiments, the tissues and/or organs generated in accordance with the
methods described herein may be transplanted in a non-human animal, and the effect of a drug
on said tissue or organ in the non-human animal may be assessed by administering the drug to a
first non-human animal that has undergone such a transplant and administering a control (e.g., a
composition that does not comprise the drug) to a second non-human animal that has undergone
such a transplant, and comparing the results.
In certain embodiments, the tissues and/or organs generated in accordance with the
methods described herein may be transplanted in a non-human animal, and the effect of a
surgical procedure on said tissue or organ in the non-human animal may be assessed by
performing the surgical procedure on the transplanted tissue/organ of a first non-human animal
that has undergone such a transplant and not performing the surgical procedure on a second non-
human animal that has undergone such a transplant, and comparing the results.
In a specific embodiment, the tissues and/or organs generated in accordance with the
methods described herein are used for extracorporeal purposes.  For example, a tissue or organ
generated in accordance with the methods described herein is situated outside of a subject's body
yet performs a function from which the subject benefits, e.g., the tissue or organ performs a
function normally performed by a tissue or organ that is situated inside a subject's body.

4.4     KITS

Provided herein is a pharmaceutical pack or kit comprising one or more containers
filled with one or more of the ingredients of the compositions described herein.  Optionally
associated with such container(s) can be a notice in the form prescribed by a governmental
agency regulating the manufacture, use or sale of pharmaceuticals or biological products, which
notice reflects approval by the agency of manufacture, use or sale for human administration.
In a specific embodiment, a kit provided herein comprises a composition comprising
the cells described herein and the flowable ECM described herein.  Such a kit may optionally
comprise a composition comprising one or more additional components (e.g., a cross-linker).  In
another specific embodiment, a kit provided herein comprises a composition comprising the cells
described herein, the flowable ECM described herein, and one or more cross-linkers described
herein.  The kits encompassed herein can be used in accordance with the methods described
herein.

5.	EXAMPLES

5.1	EXAMPLE 1: BIOPRINTED SCAFFOLDS SUPPORT ATTACHMENT
AND GROWTH OF PLACENTAL STEM CELLS

This example demonstrates that synthetic material can be bioprinted to produce
scaffolds of controlled fiber diameter and pore size, and that such scaffolds provide a suitable
substrate for the application of extracellular matrix (ECM). This example further demonstrates
that scaffolds comprising bioprinted synthetic material and ECM (hybrid scaffolds) represent a
suitable substrate for the attachment and growth of cells, including placental cells, such as
placental stem cells.

5.1.1	Methods

To fabricate hybrid scaffolds comprising synthetic material and ECM,
polycaprolactone (PCL) (Mn 45,000, Sigma) was first printed into scaffolds (54 x 54 x 0.64 mm)
using a bioprinter (EnvisionTEC, Gladbeck, Germany). The printing conditions were as follows:
temperature at 90°C, printing pressure 3~5.5 bar, printing speed 2~6 mm/s, with suitable size
needles. ECM was isolated from human placenta as previously described (see, e.g., Bhatia MB,
Wounds 20, 29, 2008). Isolated ECM was applied to both sides of the bioprinted PCL scaffolds
and allowed to dry (dehydrate) so as to generate hybrid scaffolds comprising PCL and ECM.
The resultant hybrid PCL-ECM scaffolds were punched into 10 mm diameter disks, pre-wet with
media overnight, and seeded with placental stem cells prepared in accordance with the methods
described herein (see, e.g., Section 4.1.1) at 12,500 cells/cm². The cells were cultured over an 8-
day time period. Calcein staining and MTS proliferation assays were performed in accordance
with standard protocols at different time points (n=3) to determine cell viability and proliferation.

5.1.2	Results

By optimizing printing conditions, PCL scaffolds of different fiber sizes, pore sizes
and pore structures were generated (Fig. 1). The printed fibers formed a stable network for the
generation of hybrid scaffolds comprising PCL and ECM. Further, the printing of varying fiber
sizes and pore structures made it possible to make hybrid scaffolds comprising various
properties.

Dehydration of ECM on both sides of the bioprinted PCL scaffolds resulted in the
generation of hybrid scaffolds. Good integration was seen between the PCL and ECM; no

separation between the PCL and ECM was noticed when the hybrid scaffolds were manipulated
by processing or culturing of the scaffolds, which included rehydration (Fig. 2).
The placental stem cells spread over the surface of the hybrid scaffolds over time, and
covered the majority of the surface of the hybrid scaffolds by day 6 of culture. The MTS cell
proliferation assay demonstrated that cell number significantly increased over time (Fig. 3). In
addition, the placental stem cells seeded on the hybrid scaffolds demonstrated good viability over
the 8 day culture period, as indicated by calcein staining (Fig. 4). Together, these data indicate
that PCL-ECM hybrid scaffolds support cellular attachment, survival, and growth.
5.1.3       Conclusion
This example demonstrates that hybrid scaffolds comprising ECM and synthetic
material (PCL) can be generated by methods that comprise bioprinting, and that cells not only
attach to such scaffolds, but survive and proliferate when cultured on such scaffolds.

5.2       EXAMPLE 2: BIOPRINTED SCAFFOLDS SUPPORT ATTACHMENT
AND GROWTH OF PLACENTAL STEM CELLS
This example demonstrates that synthetic material and ECM comprising cells, such as
placental cells, e.g., placental stem cells, can be simultaneously bioprinted to produce hybrid
scaffolds. As demonstrated by this Example, the bioprinted cells not only survive the bioprinting
process, but proliferate over time in culture with the hybrid scaffolds.
5.2.1       Methods
ECM was prepared as described in Example 1 and mixed with 0.5% alginate hydrogel
containing 1 million/ml placental stem cells. Next, PCL and the cell-containing ECM were
bioprinted, in layers, to generate a hybrid scaffold comprising PCL and ECM. In each layer of
the scaffold, PCL was first printed, then the ECM/cell component was printed to fill the gaps in
between the PCL lines. Two or five of such layers were printed and crosslinked with CaCl2
solution to generate the hybrid scaffolds. The bioprinted, cell-containing scaffolds
(cells/ECM/PCL) were cultured for seven days, and cell proliferation and survival were assessed
at various time points via calcein staining and an MTS cell proliferation assay.
5.2.2       Results
The bioprinted scaffolds maintained an intact structure throughout the duration of cell
culture (Fig. 5). PCL provided a good structural support for the ECM hydrogels, which allowed

for the generation of three-dimensional constructs. Following bioprinting and throughout
culture, the cells were well-distributed throughout the three-dimensional constructs; cells were
found throughout the depth of the scaffolds during culture (Fig. 6).
The placental stem cells survived the bioprinting process and continued to proliferate
in the three-dimensional bioprinted hybrid scaffolds throughout culture, as evidenced by calcein
staining (Fig. 7). As shown in Fig. 8, most of the cells were found to spread throughout the
ECM in the hybrid scaffolds, indicating that the ECM enhanced cell attachment and spreading in
the ECM hydrogel. This was confirmed by comparing the location of cells in alginate alone with
that of the cells in the scaffolds. Additionally, as shown in Figure 9, an MTS cell proliferation
assay demonstrated increases in cell number for both the 2-layer and 5-layer scaffolds, indicating
that these hybrid scaffolds supported cell growth.
5.2.3 Conclusion
This example demonstrates that hybrid scaffolds comprising ECM and synthetic
material (PCL) can be generated by methods that comprise simultaneous bioprinting of ECM and
PCL. Also demonstrated by this Example is the fact that cells can be bioprinted along with the
components of the hybrid scaffold (ECM and PCL), and that the cells survive the bioprinting
process. Further, the cells bioprinted along with the components of the hybrid scaffold
proliferate when cultured on such scaffolds and intersperse throughout the scaffolds better than
when cultured in cellular matrix (alginate) alone.

5.3 EXAMPLE 3: FUNCTIONAL BIOPRINTED SCAFFOLDS
This example demonstrates that synthetic material and ECM comprising cells can be
bioprinted to produce functional scaffolds.
ß-TC-6 cells, an insulin producing cell line, were bioprinted with human placenta
derived extracellular matrix (ECM) into a bioprinted scaffold. The scaffold was 15 x 15 x 2.5
mm in dimensions, and contained 5 layers. In each layer, polycaprolactone (PCL) was first
printed, followed by printing of ß-TC-6 cells, mixed at 15 million cells/ml in alginate-ECM
hydrogel (1% alginate and 12% ECM) between the PCL lines. The entire scaffold was immersed
in 1% calcium chloride solution to crosslink for 20 minutes. The scaffolds then were cultured in
DMEM medium containing 15% fetal calf serum in a cell culture incubator in 6 well plates (3 to
5 ml of medium per well). At different time points, the scaffolds were harvested for calcein

staining and MTS proliferation assays, to characterize cell viability and cell proliferation,
respectively. Figure 10 shows the structure of the bio-printed scaffolds.
Calcein staining demonstrated that the ß-TC-6 cells survived the printing process and
remained viable during culture. A cross-sectional view of the scaffolds showed that the cells
distributed evenly throughout the scaffolds, and remained alive in each layer (see Figure 10).
The MTS assay confirmed that the insulin producing ß-TC-6 cells remained viable for up to 3
weeks, with the overall number of viable cells remaining constant (see Figure 11).
To determine whether the ß-TC-6 cells could function in the bioprinted scaffold,
insulin production by the cells was measured. To measure insulin production, the bio-printed
scaffolds were exposed to fresh growth medium (3 ml/well in a 6-well plate) for 2 hours and
aliquots of the supernatant from each scaffold were measured for insulin concentration using a
mouse insulin ELISA kit (Millipore). The highest level of insulin produced was detected at day
0 (see Figure 12). The levels of secreted insulin decreased in culture afterwards (day-3 and day-
6) but remained stable from day 3 to day 6 in the culture (see Figure 12). Thus, the ß-TC-6 cells
maintained the ability to produce and secrete insulin after being bioprinted.
A key function of insulin producing cells in the pancreas is to produce insulin in
response to increased glucose levels in the blood. It was thus examined whether the bioprinted
scaffolds comprising PCL, ECM, and ß-TC-6 cells retained this function by exposing the
scaffolds to a glucose surge challenge (see Figure 13). One scaffold ("A" of Figure 13) was
exposed to glucose starvation conditions (IMDM medium without glucose, 10% FCS) for two
days and then challenged with an insulin producing condition (50 mM glucose/1 mM IBMX).
As controls, bioprinted scaffolds were maintained in normal culture medium with steady glucose
levels ("B" and "C" of Figure 13). In the controls, the medium was changed at the same time
that the challenge with an insulin producing condition was performed for the test scaffold (i.e., A
of Figure 13). The supernatant from each culture (A, B, and C) was sampled every half hour and
the insulin concentration from each supernatant was measured by ELISA. Figure 13 shows the
levels of insulin production from each culture at the different time points and demonstrates that
the bioprinted scaffold exposed to glucose starvation conditions followed by challenge with an
insulin producing condition (i.e., A of Figure 13) produced greater than 80-fold more insulin
after 3 hours after challenge as compared to its level of insulin production at 0.5 hours post-
challenge, while the controls (i.e., B and C of Figure 13) produced much less insulin (only

approximately 2-fold more insulin after 3 hours following media change as compared to the level
of insulin production at 0.5 hour post-media change).
This Example demonstrates that bioprinted scaffolds comprising synthetic material,
cells, and ECM can be generated and that the cells of the bioprinted scaffolds remain both viable
and functional.

The compositions and methods disclosed herein are not to be limited in scope by the
specific embodiments described herein.  Indeed, various modifications of the compositions and
methods in addition to those described will become apparent to those of skill in the art from the
foregoing description and accompanying figures.  Such modifications are intended to fall within
the scope of the appended claims.
Various publications, patents and patent applications are cited herein, the disclosures
of which are incorporated by reference in their entireties.

The following numbered paragraphs (paras.) contain further statements of various aspects of the
present invention:-

1.	A method of forming a three-dimensional tissue or organ comprising depositing at least
one cellular composition comprising cells and extracellular matrix (ECM) onto a surface.

2.	The method of para 1, wherein said ECM comprises flowable ECM.

3.	The method of para 1 or 2, wherein said cellular composition and said ECM are printed
onto said surface.

4.	The method of any one of paras 1-3, wherein said depositing is accomplished by inkjet
printing.

5.	The method of any of paras 1-4, wherein said surface is an artificial surface.

6.	The method of any of paras 1-4, wherein said surface is decellularized tissue or a
decellularized organ.

7.	The method of any of paras 1-6, wherein said ECM comprises mammalian ECM,
molluscan ECM, piscene ECM, and or plant ECM.

8.	The method of para 7, wherein said mammalian ECM is placental ECM.

9.	The method of para 8, wherein said ECM comprises telopeptide placental collagen.

10.	The method of para 9, wherein said telopeptide placental collagen comprises base-
treated, detergent treated Type I telopeptide placental collagen.

11.	The method of para 9 or 10, wherein said collagen has not been chemically modified or
contacted with a protease.

12.	The method of para 8, wherein said placental ECM comprises base-treated and/or
detergent treated Type I telopeptide placental collagen that has not been chemically modified or
contacted with a protease, wherein said ECM comprises less than 5% fibronectin or less than 5%
laminin by weight; between 25% and 92% Type I collagen by weight; and 2% to 50% Type III
collagen or 2% to 50% type IV collagen by weight.

13.	The method of para 8, wherein said placental ECM comprises base-treated, detergent
treated Type I telopeptide placental collagen that has not been chemically modified or contacted

with a protease, wherein said ECM comprises less than 1% fibronectin or less than 1% laminin
by weight; between 74% and 92% Type I collagen by weight; and 4% to 6% Type III collagen or
2% to 15% type IV collagen by weight.
14.	The method of any one of paras 1-13, wherein said ECM is derivatized prior to said
deposition.
15.	The method of para 14, wherein said ECM is derivatized with one or more of a cell
attachment peptide, a cell attachment protein, a cytokine, or a glycosaminoglycan.
16.	The method of any of paras 1-15, further comprising deposition of a cell attachment
peptide, a cell attachment protein, a cytokine, or a glycosaminoglycan.
17.	The method of para 16, wherein said cytokine is vascular endothelial growth factor
(VEGF), or a bone morphogenetic protein (BMP).
18.	The method of para 16, wherein said cell attachment peptide is a peptide comprising one
or more RGD motifs.
19.	The method of any of paras 1-18, further comprising deposition of a synthetic polymer.
20.	The method of para 19, wherein said synthetic polymer is thermosensitive.
21.	The method of para 19, wherein said synthetic polymer is photosensitive.
22.	The method of para 19, wherein said synthetic polymer comprises a thermoplastic.
23.	The method of para 22, wherein said synthetic polymer is poly(L-lactide-co-glycolide)
(PLGA).
24.	The method of para 22, wherein said thermoplastic is polycaprolactone, polylactic acid,
polybutylene terephthalate, polyethylene terephthalate, polyethylene, polyester, polyvinyl acetate,
or polyvinyl chloride.
25.	The method of para 19, wherein said synthetic polymer is polyacrylamide,
polyvinylidine chloride, poly(o-carboxyphenoxy)-p-xylene) (poly(o-CPX)), poly(lactide-
anhydride) (PLAA), n-isopropyl acrylamide, pent erythritol diacrylate, polymethyl acrylate,
carboxymethylcellulose, or poly(lactic-co-glycolic acid) (PLGA).

26.	The method of any of paras 1-25, further comprising deposition of tenascin C or a
fragment thereof.
27.	The method of any of paras 1-35, further comprising deposition of a titanium-
aluminum-vanadium (Ti6Al4V) composition.
28.	The method of para 27, wherein said titanium-aluminum-vanadium composition is
deposited in the form of an interconnected porous network of fibers.
29.	The method of para 1, wherein said tissue or organ comprises at least one layer of said
cellular composition and at least one layer of ECM.
30.	The method of para 1, wherein at least a portion of said tissue or organ comprises at
least one layer of said cellular composition and at least one layer of said ECM printed in
alternating layers.
31.	The method of para 19, wherein in producing said tissue or organ, at least a portion of
said synthetic polymer is printed in the form of a plurality of fibers that are substantially parallel
to each other.
32.	The method of para 31, wherein said plurality of fibers are deposited substantially in
parallel to each other are printed so as not to physically contact each other.
33.	The method of para 32, wherein said synthetic polymer is deposited a plurality of times.
34.	The method of para 33, wherein said fibers, when deposited said plurality of times, are
printed in a different orientation in at least two of said times.
35.	The method of para 33, wherein said fibers, when deposited said plurality of times, are
deposited in a different orientation in each of said times.
36.	The method of any of paras 1-35, wherein said tissue comprises at least two layers of
said ECM.
37.	The method of para 36, wherein at least a portion of said at least two layers of said
substrate are separated from each other by said cellular composition.
38.	The method of para 3, wherein said printing comprises printing an adhesive between
said two layers of substrate.

39.	The method of any of paras 1-38, wherein said ECM and said cellular composition are
deposited onto said surface separately.
40.	The method of any of paras 1-38, wherein at least a portion of said ECM is deposited
onto said surface prior to printing said cellular composition.
41.	The method of any of paras 1-38, wherein said cellular composition and said ECM are
combined prior to said depositing.
42.	The method of para 3, wherein said cellular composition is printed onto said ECM
during said printing.
43.	The method of para 3, wherein said cellular composition is printed onto said ECM after
completion of said printing of said ECM.
44.	The method of any of paras 1-43, wherein said ECM is formed into a three-dimensional
structure during said depositing.
45.	The method of any of paras 1-44, further comprising deposition of a bone substitute.
46.	The method of para 1-45, wherein said surface is or comprises a bone.
47.	The method of para 45, wherein said bone substitute is printed to correspond to a bone
in an intended recipient of said tissue.
48.	The method of para 47, wherein the method further comprises generating a three-
dimensional map of a bone in an intended recipient of said tissue, wherein said bone substitute is
printed to correspond to said three-dimensional map.
49.	The method of para 47 or para 48, wherein said bone is a cranial bone or a facial bone.
50.	The method of para 47 or para 48, wherein said bone is an otic bone or a bone of the
phalanges.
51.	The method of any of paras 47-50, wherein said cellular composition is printed on said
bone or bone substitute such that said cellular composition at least partially covers the surface of
said bone or bone substitute.
52.	The method of para 1, wherein said tissue comprises (a) a surface consisting of a bone
having an inner face and an outer face, and (b) two cellular compositions, wherein said first

cellular composition comprises a first type of cell that is printed on said inner face, and a second
type of cell that is printed on said outer face.

53.	The method of any of paras 1-52, wherein said deposition is performed three-
dimensionally.

54.	The method of para 53, wherein said tissue is printed onto a three-dimensional surface.

55.	The method of para 4, wherein said inkjet printing is performed using a printer with a
plurality of print heads or a plurality of print jets.

56.	The method of para 55, wherein each of said print heads or print jets is separately
controllable.

57.	The method of para 56, wherein each of said print heads or print jets operates
independently from the remaining said print heads or print jets.

58.	The method of any of paras 55-57, wherein at least one of said plurality of print heads or
print jets prints said cellular composition, and at least one other of said plurality of print heads or
print jets prints said ECM.

59.	The method of para 1, wherein said cellular composition and said ECM are combined
prior to said printing.

60.	The method of any of paras 1-59, wherein said cellular composition comprises bone
marrow-derived mesenchymal stem cells (BM-MSCs).

61.	The method of any of paras 1-59, wherein said cellular composition comprises tissue
culture plastic-adherent CD34-, CDIO+, CD105+, CD200+ placental stem cells.

62.	The method of para 61, wherein said placental stem cells are additionally one or more of
CD45-, CD80-, CD86-, or CD90+.

63.	The method of para 61 or 62, wherein said placental stem cells suppress an immune
response in said recipient.

64.	The method of para 63, wherein said placental stem cells suppresses an immune
response locally within said recipient.

65.	The method of any of paras 1-59, wherein said cellular composition comprises
embryonic stem cells, embryonic germ cells, induced pluripotent stem cells, mesenchymal stem
cells, bone marrow-derived mesenchymal stem cells, bone marrow-derived mesenchymal
stromal cells, tissue plastic-adherent placental stem cells (PDACs), umbilical cord stem cells,
amniotic fluid stem cells, amnion derived adherent cells (AMDACs), osteogenic placental
adherent cells (OPACs), adipose stem cells, limbal stem cells, dental pulp stem cells, myoblasts,
endothelial progenitor cells, neuronal stem cells, exfoliated teeth derived stem cells, hair follicle
stem cells, dermal stem cells, parthenogenically derived stem cells, reprogrammed stem cells,
amnion derived adherent cells, or side population stem cells.

66.	The method of any of paras 1-59, wherein said cellular composition comprises
differentiated cells.

67.	The method of para 66, wherein said differentiated cells comprise endothelial cells,
epithelial cells, dermal cells, endodermal cells, mesodermal cells, fibroblasts, osteocytes,
chondrocytes, natural killer cells, dendritic cells, hepatic cells, pancreatic cells, or stromal cells.

68.	The method of para 66, wherein said differentiated cells comprise salivary gland
mucous cells, salivary gland serous cells, von Ebner's gland cells, mammary gland cells, lacrimal
gland cells, ceruminous gland cells, eccrine sweat gland dark cells, eccrine sweat gland clear
cells, apocrine sweat gland cells, gland of Moll cells, sebaceous gland cells. bowman's gland
cells, Brunner's gland cells, seminal vesicle cells, prostate gland cells, bulbourethral gland cells,
Bartholin's gland cells, gland of Littre cells, uterus endometrium cells, isolated goblet cells,
stomach lining mucous cells, gastric gland zymogenic cells, gastric gland oxyntic cells,
pancreatic acinar cells, paneth cells, type II pneumocytes, clara cells,

somatotropes, lactotropes, thyrotropes, gonadotropes, corticotropes, intermediate pituitary cells,
magnocellular neurosecretory cells, gut cells, respiratory tract cells, thyroid epithelial cells,
parafollicular cells, parathyroid gland cells, parathyroid chief cell, oxyphil cell, adrenal gland
cells, chromaffin cells, Leydig cells, theca intema cells, corpus luteum cells, granulosa lutein
cells, theca lutein cells, juxtaglomerular cell, macula densa cells, peripolar cells, mesangial cell,

blood vessel and lymphatic vascular endothelial fenestrated cells, blood vessel and lymphatic
vascular endothelial continuous cells, blood vessel and lymphatic vascular endothelial splenic
cells, synovial cells, serosal cell (lining peritoneal, pleural, and pericardial cavities), squamous

cells, columnar cells, dark cells, vestibular membrane cell (lining endolymphatic space of ear),
stria vascularis basal cells, stria vascularis marginal cell (lining endolymphatic space of ear),
cells of Claudius, cells of Boettcher, choroid plexus cells, pia-arachnoid squamous cells,
pigmented ciliary epithelium cells, nonpigmented ciliary epithelium cells, corneal endothelial
cells, peg cells,

respiratory tract ciliated cells, oviduct ciliated cell, uterine endometrial ciliated cells, rete testis
ciliated cells, ductulus efferens ciliated cells, ciliated ependymal cells,

epidermal keratinocytes, epidermal basal cells, keratinocyte of fingernails and toenails, nail bed
basal cells, medullary hair shaft cells, cortical hair shaft cells, cuticular hair shaft cells, cuticular
hair root sheath cells, hair root sheath cells of Huxley's layer, hair root sheath cells of Henle's
layer, external hair root sheath cells, hair matrix cells,

surface epithelial cells of stratified squamous epithelium, basal cell of epithelia, urinary
epithelium cells,

auditory inner hair cells of organ of Corti, auditory outer hair cells of organ of Corti, basal cells
of olfactory epithelium, cold-sensitive primary sensory neurons, heat-sensitive primary sensory
neurons, Merkel cells of epidermis, olfactory receptor neurons, pain-sensitive primary sensory
neurons, photoreceptor rod cells, photoreceptor blue-sensitive cone cells, photoreceptor green-
sensitive cone cells, photoreceptor red-sensitive cone cells, proprioceptive primary sensory
neurons, touch-sensitive primary sensory neurons, type I carotid body cells, type II carotid body
cell (blood pH sensor), type I hair cell of vestibular apparatus of ear (acceleration and gravity),
type II hair cells of vestibular apparatus of ear, type I taste bud cells

cholinergic neural cells, adrenergic neural cells, peptidergic neural cells,

inner pillar cells of organ of Corti, outer pillar cells of organ of Corti, inner phalangeal cells of
organ of Corti, outer phalangeal cells of organ of Corti, border cells of organ of Corti, Hensen
cells of organ of Corti, vestibular apparatus supporting cells, taste bud supporting cells, olfactory
epithelium supporting cells, Schwann cells, satellite cells, enteric glial cells,

astrocytes, neurons, oligodendrocytes, spindle neurons,

anterior lens epithelial cells, crystallin-containing lens fiber cells,

hepatocytes, adipocytes, white fat cells, brown fat cells, liver lipocytes,

kidney glomerulus parietal cells, kidney glomerulus podocytes, kidney proximal tubule brush
border cells, loop of Henle thin segment cells, kidney distal tubule cells, kidney collecting duct
cells, type I pneumocytes, pancreatic duct cells, nonstriated duct cells, duct cells, intestinal brush
border cells, exocrine gland striated duct cells, gall bladder epithelial cells, ductulus efferens
nonciliated cells, epididymal principal cells, epididymal basal cells,

ameloblast epithelial cells, planum semilunatum epithelial cells, organ of Corti interdental
epithelial cells, loose connective tissue fibroblasts, corneal keratocytes, tendon fibroblasts, bone
marrow reticular tissue fibroblasts, nonepithelial fibroblasts, pericytes, nucleus pulposus cells,
cementoblast/cementocytes, odontoblasts, odontocytes, hyaline cartilage chondrocytes,
fibrocartilage chondrocytes, elastic cartilage chondrocytes, osteoblasts, osteocytes, osteoclasts,
osteoprogenitor cells, hyalocytes, stellate cells (ear), hepatic stellate cells (Ito cells), pancreatic
stelle cells,

red skeletal muscle cells, white skeletal muscle cells, intermediate skeletal muscle cells, nuclear
bag cells of muscle spindle, nuclear chain cells of muscle spindle, satellite cells, ordinary heart
muscle cells, nodal heart muscle cells, Purkinje fiber cells, mooth muscle cells, myoepithelial
cells of iris, myoepithelial cell of exocrine glands,

reticulocytes, megakaryocytes, monocytes, connective tissue macrophages. epidermal
Langerhans cells, dendritic cells, microglial cells, neutrophils, eosinophils, basophils, mast cell,
helper T cells, suppressor T cells, cytotoxic T cell, natural Killer T cells, B cells, natural killer
cells,

melanocytes, retinal pigmented epithelial cells,

oogonia/oocytes, spermatids, spermatocytes, spermatogonium cells, spermatozoa, ovarian
follicle cells, Sertoli cells, thymus epithelial cell, and/or interstitial kidney cells.

69.	The method of any of paras 1-68, wherein said tissue comprises a nerve guidance
conduit.

70.	The method of para 69, wherein said nerve guidance conduit is made of a polyanhydride.

71.	The method of para 70, wherein said polyanhydride is poly(o-carboxyphenoxy)-p-
xylene) or poly(lactide-anhydride).

72.	The method of any of paras 69-71, wherein said nerve guidance conduit is deposited
using said polyanhydride into said tissue by said inkjet printing.
73.	The method of any of paras 69-71, wherein said nerve guidance conduit is prepared
prior to said printing, and is placed into said tissue during printing of said tissue.
74.	The method of para 72 or para 73, wherein said tissue comprising said nerve guidance
conduit is suitable for implantation into a damaged area of the central nervous system (CNS).
75.	The method of para 74, wherein said area of the CNS is the spinal cord.

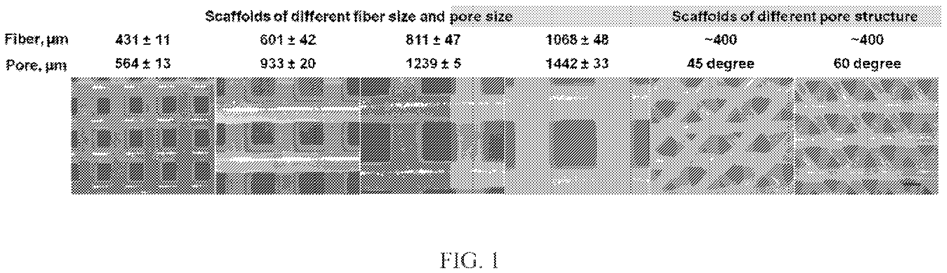

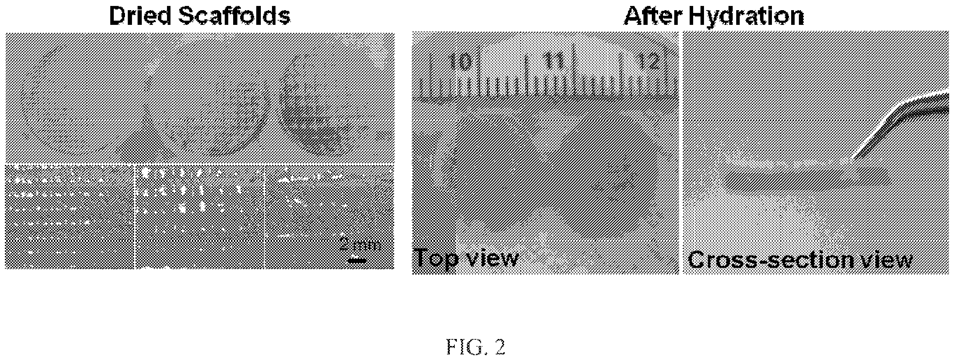

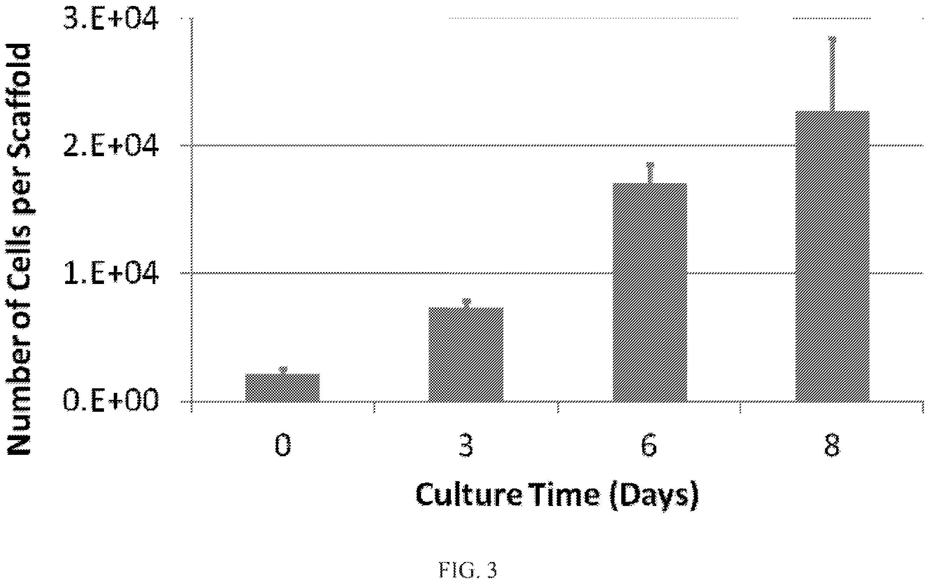

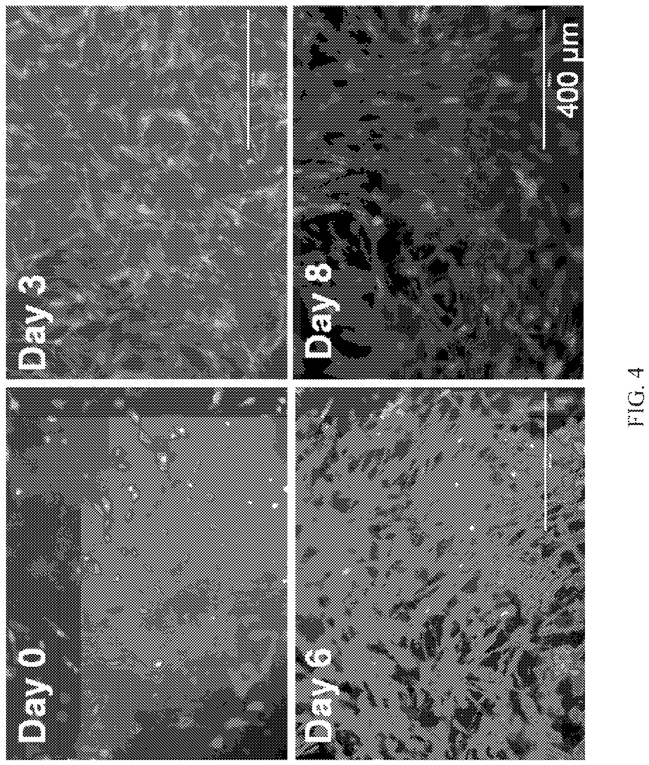

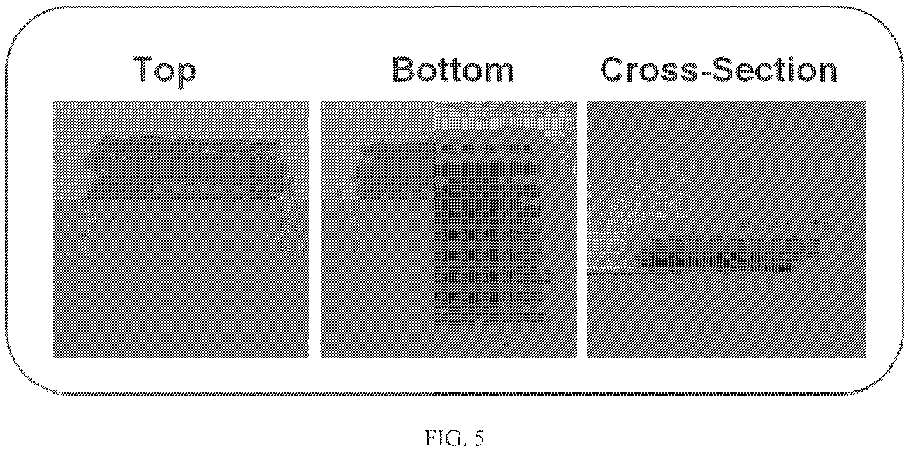

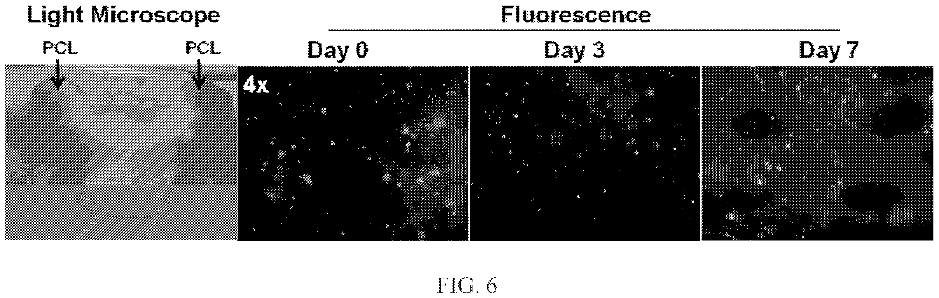

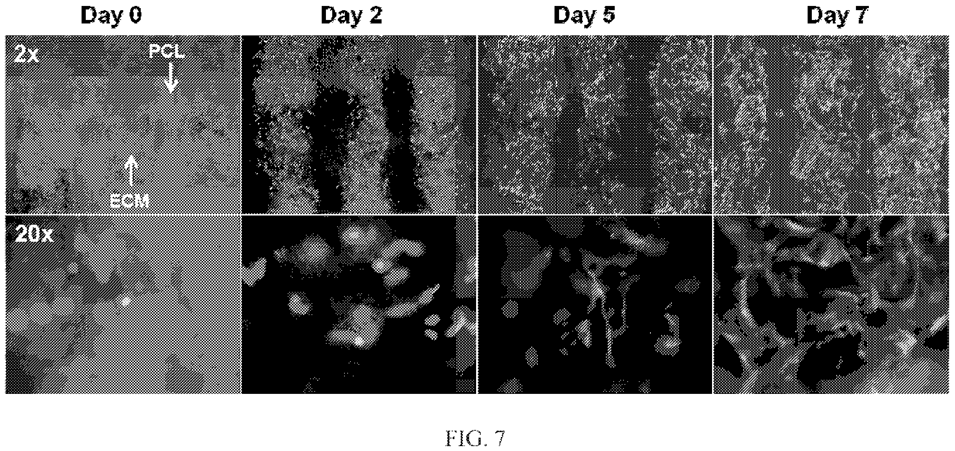

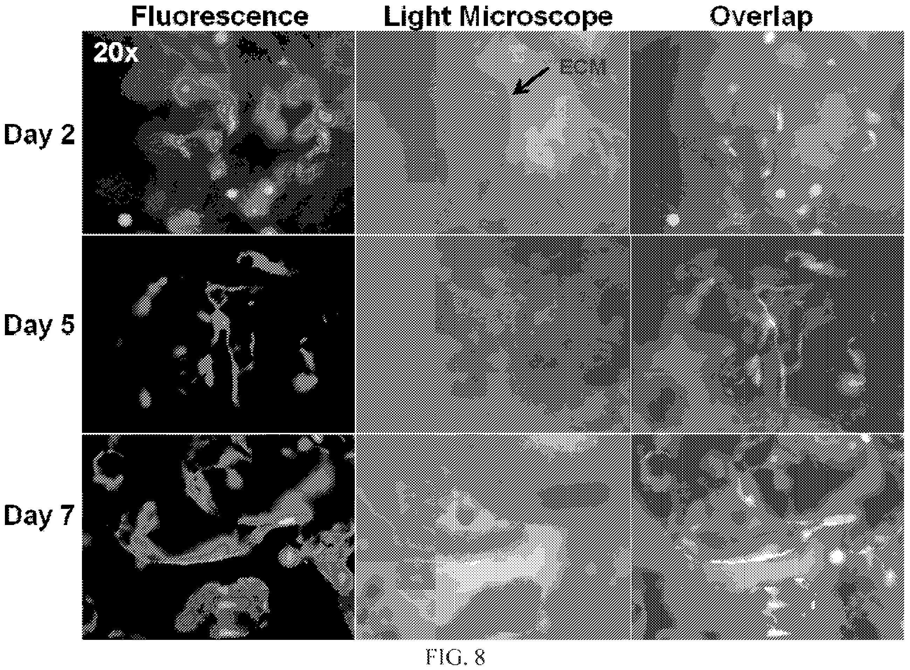

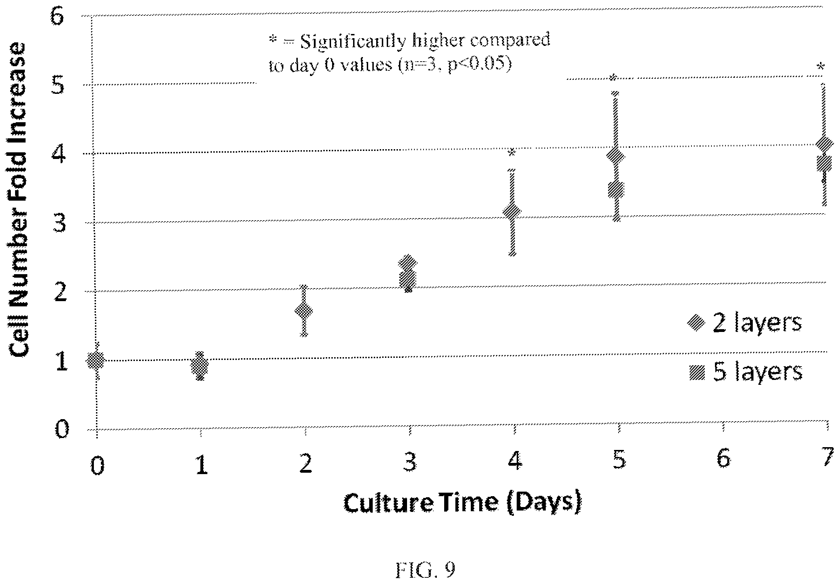

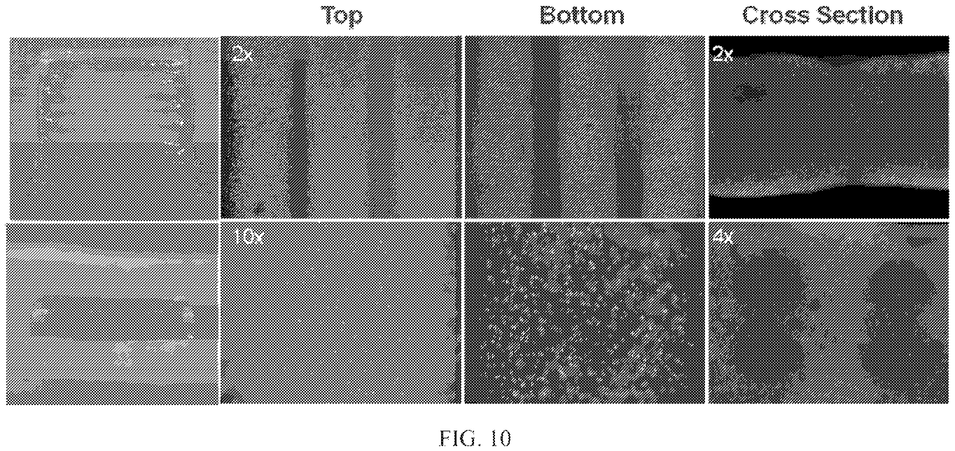

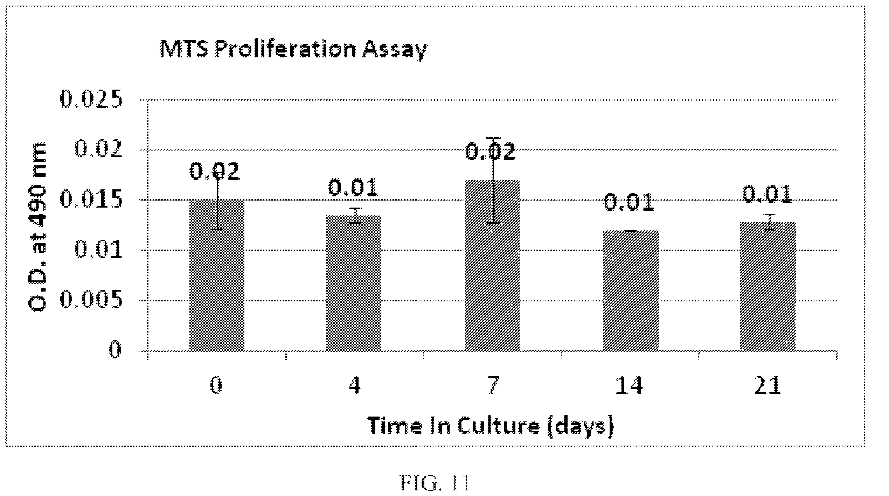

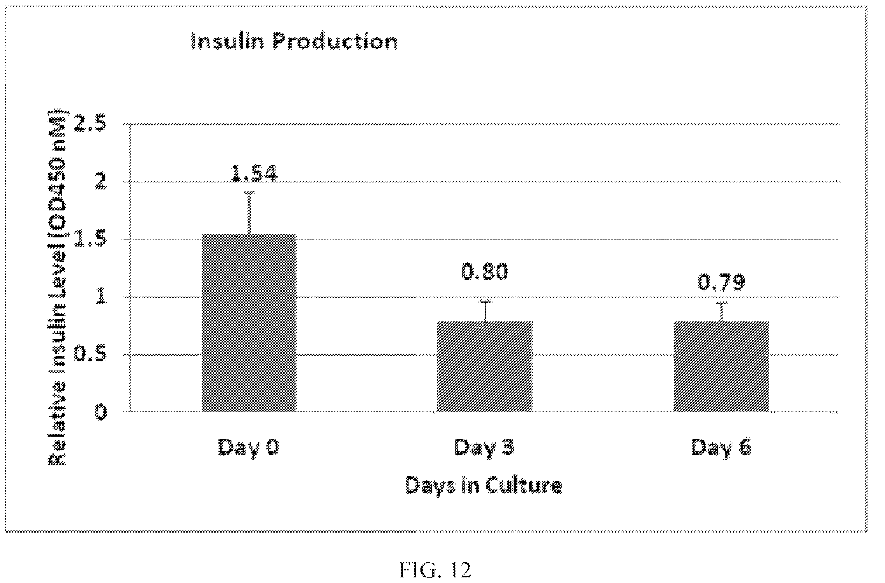

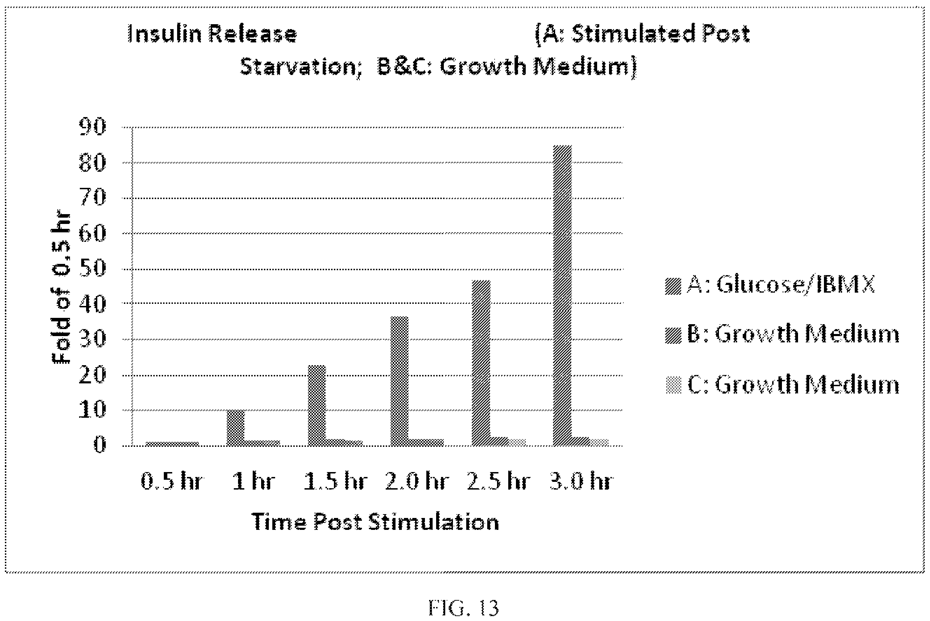

## Claims

1. A method of forming a three-dimensional tissue or organ comprising depositing at least one cellular composition comprising cells and extracellular matrix (ECM) onto a surface.

2. The method of claim 1, wherein said ECM comprises flowable ECM.

3. The method of claim 1 or 2, wherein said cellular composition and said ECM are printed onto said surface, preferably wherein said depositing is accomplished by inkjet printing.

4. The method of any of claims 1-3, wherein (i) said surface is an artificial surface; or (ii) said surface is decellularized tissue or a decellularized organ.

5. The method of any of claims 1-4, wherein said ECM is placental ECM.

6. The method of any of claims 1-5, further comprising deposition of a cell attachment peptide, a cell attachment protein, a cytokine, a glycosaminoglycan, a synthetic polymer, tenascin C or a fragment thereof, and/or a titanium-aluminum-vanadium (Ti₆Al₄V) composition.

7. The method of claim 6, wherein (i) said cytokine is vascular endothelial growth factor (VEGF), or a bone morphogenetic protein (BMP); (ii) said cell attachment peptide is a peptide comprising one or more RGD motifs; (iii) said synthetic polymer is thermosensitive or photosensitive; (iv) said synthetic polymer comprising a thermoplastic; (v) said synthetic polymer is polyacrylamide, polyvinylidine chloride, poly(o-carboxyphenoxy)-p-xylene) (poly(o-CPX)), poly(lactide-anhydride)(PLAA), n-isopropyl acrylamide, pent erythritol diacrylate, polymethyl acrylate, carboxymethylcellulose, or poly(lactic-co-glycolic acid) (PLGA); and/or said titanium-aluminum-vanadium composition is deposited in the form of an interconnected porous network of fibers.

8. The method of claim 1,
(i) wherein said tissue or organ comprises at least one layer of said cellular composition and at least one layer of ECM;
(ii) wherein at least a portion of said tissue or organ comprises at least one layer of said cellular composition and at least one layer of said ECM printed in alternating layers; or
(iii) wherein said tissue comprises at least two layers of said ECM.

9. The method of claim 8 (iii), wherein at least a portion of said at least two layers of said substrate are separated from each other by said cellular composition.

10. The method of claim 3, wherein said printing comprises printing an adhesive between said two layers of substrate.

11. The method claim 1,
wherein said ECM and said cellular composition are deposited onto said surface separately;
wherein at least a portion of said ECM is deposited onto said surface prior to printing said cellular composition; or
wherein said cellular composition and said ECM are combined prior to said depositing.

12. The method of claim 3, wherein said cellular composition is printed onto said ECM during said printing or after completion of said printing of said ECM.

13. The method of claim 1, wherein said ECM is formed into a three-dimensional structure during said depositing.

14. The method of claim 1, further comprising deposition of a bone substitute, preferably, wherein said surface is or comprises a bone.

15. The method of claim 1, wherein said tissue comprises (a) a surface consisting of a bone having an inner face and an outer face, and (b) two cellular compositions, wherein said first cellular composition comprises a first type of cell that is printed on said inner face, and a second type of cell that is printed on said outer face, preferably, wherein said tissue is printed onto a three-dimensional surface.

16. The method of any of claims 1-15, wherein said cellular composition comprises (i) bone marrow-derived mesenchymal stem cells (BM-MSCs); (ii) tissue culture plastic-adherent CD34-, CD10+, CD105+, CD200+ placental stem cells; (iii) embryonic stem cells, embryonic germ cells, induced pluripotent stem cells, mesenchymal stem cells, bone marrow-derived mesenchymal stem cells, bone marrow-derived mesenchymal stromal cells, tissue plastic-adherent placental stem cells (PDACs), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells; and/or (iv) differentiated cells.

17. The method of claim16, wherein said differentiated cells comprise endothelial cells, epithelial cells, dermal cells, endodermal cells, mesodermal cells, fibroblasts, osteocytes, chondrocytes, natural killer cells, dendritic cells, hepatic cells, pancreatic cells, stromal cells;
salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells, bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, clara cells,
somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca interna cells, corpus luteum cells, granulosa lutein cells, theca lutein cells, juxtaglomerular cell, macula densa cells, peripolar cells, mesangial cell,
blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells,
respiratory tract ciliated cells, oviduct ciliated cell, uterine endometrial ciliated cells, rete testis ciliated cells, ductulus efferens ciliated cells, ciliated ependymal cells,
epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells,
surface epithelial cells of stratified squamous epithelium, basal cell of epithelia, urinary epithelium cells,
auditory inner hair cells of organ of Corti, auditory outer hair cells of organ of Corti, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells
cholinergic neural cells, adrenergic neural cells, peptidergic neural cells,
inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells,
astrocytes, neurons, oligodendrocytes, spindle neurons,
anterior lens epithelial cells, crystallin-containing lens fiber cells,
hepatocytes, adipocytes, white fat cells, brown fat cells, liver lipocytes,
kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, epididymal basal cells,
ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells,
red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, mooth muscle cells, myoepithelial cells of iris, myoepithelial cell of exocrine glands,
reticulocytes, megakaryocytes, monocytes, connective tissue macrophages, epidermal Langerhans cells, dendritic cells, microglial cells, neutrophils, eosinophils, basophils, mast cell, helper T cells, suppressor T cells, cytotoxic T cell, natural Killer T cells, B cells, natural killer cells,
melanocytes, retinal pigmented epithelial cells,
oogonia/oocytes, spermatids, spermatocytes, spermatogonium cells, spermatozoa, ovarian follicle cells, Sertoli cells, thymus epithelial cell, and/or interstitial kidney cells.

18. The method of any of claims 1-17, wherein said tissue comprises a nerve guidance conduit.
